# EUROPÄISCHE PATENTSCHRIFT

(11) **EP 1 633 478 B1**
(45) Veröffentlichungstag und Bekanntmachung des Hinweises auf die Patenterteilung: **13.07.2011**
(21) Anmeldenummer: 04735006.1
(22) Anmeldetag: 27.05.2004
(51) Int. Cl.: B01J 37/08, B01J 23/888, B01J 23/887, C07C 51/225, C07C 51/25

(54) **VERFAHREN ZUR HERSTELLUNG VON KATALYTISCH AKTIVEN MULTIELEMENTOXIDMASSEN, DIE WENIGSTENS EINES DER ELEMENTE NB UND W SOWIE DIE ELEMENTE MO, V UND CU ENTHALTEN, MITTELS THERMISCHER BEHANDLUNG IN SAUERSTOFFARMER ATMOSPHÄRE**
METHOD FOR THE PRODUCTION OF CATALYTICALLY ACTIVE MULTIELEMENT OXIDE MATERIALS CONTAINING AT LEAST ONE OF THE ELEMENTS NB AND W AS WELL AS ELEMENTS MO, V, AND CU BY MEANS OF A THERMAL TREATMENT IN A LOW-OXYGEN ATMOSPHERE
PROCEDE DE PRODUCTION DE MASSES D'OXYDES MULTIELEMENTS A ACTION CATALYTIQUE COMPRENANT L'ELEMENT NB ET/OU L'ELEMENT W, AINSI QUE LES ELEMENTS MO, V ET CU, PAR TRAITEMENT THERMIQUE DANS UNE ATMOSPHERE PAUVRE EN OXYGENE

(30) Priorität: 04.06.2003 DE 10325488; 04.06.2003 US 475488 P; 19.12.2003 US 530617 P; 19.12.2003 DE 10360058
(43) Veröffentlichungstag der Anmeldung: 15.03.2006
(73) Patentinhaber: BASF SE, 67056 Ludwigshafen (DE)
(72) Erfinder: DIETERLE, Martin, 68167 Mannheim (DE); HIBST, Hartmut, 69198 Schriesheim (DE); PÖPEL, Wolfgang, Jürgen, 64297 Darmstadt (DE); PETZOLDT, Jochen, 68163 Mannheim (DE); MÜLLER-ENGEL, Klaus, Joachim, 76297 Stutensee (DE)
(86) Internationale Anmeldenummer: PCT/EP2004/005695
(87) Internationale Veröffentlichungsnummer: WO 2004/108284

(56) Entgegenhaltungen:
- DE-A- 10 046 928
- US-A- 4 111 983
- US-A- 4 289 654
- US-A- 5 637 546

## Beschreibung

Vorliegende Erfindung betrifft ein Verfahren zur Herstellung von katalytisch aktiven Multielementoxidmassen, die wenigstens eines der Elemente Nb und W sowie die Elemente Mo, V und Cu enthalten, wobei der molare Anteil des Elementes Mo an der Gesamtmenge aller von Sauerstoff verschiedenen Elemente der katalytisch aktiven Multielementoxidmasse 20 mol-% bis 80 mol% beträgt, das molare Verhältnis von in der katalytisch aktiven Multielementoxidmasse enthaltenem Mo zu in der katalytisch aktiven Multielementoxidmasse enthaltenem V, Mo/V, 15:1 bis 1:1 beträgt, das entsprechende molare Verhältnis Mo/Cu 30:1 bis 1:3 und das entsprechende molare Verhältnis Mo/(Gesamtmenge aus W und Nb) 80:1 bis 1:4 beträgt und bei dem man aus Ausgangsverbindungen, die die von Sauerstoff verschiedenen elementaren Konstituenten der Multielementoxidmasse als Bestandteile enthalten, ein inniges, auch Ammoniumionen enthaltendes, Trockengemisch herstellt und dieses in einer an molekularem Sauerstoff armen Atmosphäre bei erhöhter Temperatur thermisch behandelt, wobei bei Temperaturen ≥160°C, wenigstens eine Teilmenge der im innigen Trockengemisch enthaltenen Ammoniumionen unter Freisetzung von Ammoniak zersetzt wird.

Außerdem betrifft vorliegende Erfindung ein Verfahren zur Herstellung von Acrylsäure durch heterogen katalysierte partielle Gasphasenoxidation von Acrolein unter Verwendung von Katalysatoren, die die vorgenannten Multielementoxidmassen als katalytisch aktive Massen enthalten.

Das eingangs beschriebene Verfahren zur Herstellung von katalytisch aktiven Multielementoxidmassen ist ebenso bekannt wie die Verwendung der dabei erhältlichen Multielementoxidmassen als Aktivmasse in Katalysatoren für die heterogen katalysierte partielle Gasphasenoxidation von Acrolein zu Acrylsäure.

Aus der DE-31 19 586 C2 ist bekannt, eine katalytisch aktive Multielementoxidmasse, die als Grundbestandteil die Elemente Mo und V enthält, dadurch herzustellen, dass man aus Ausgangsverbindungen, die die elementaren Konstituenten der Multielementoxidmassen als Bestandteile enthalten, ein inniges, Ammoniumionen umfassendes, Trockengemisch herstellt, und dieses bei 380°C in einem Gasstrom, der 1 Vol.% molekularen Sauerstoff enthält, thermisch behandelt.

Die resultierenden Multielementoxide werden als Aktivmasse für Katalysatoren zur katalytischen partiellen Gasphasenoxidation von Acrolein zu Acrylsäure empfohlen. DATABASE WPI, Week 7512, Derwent Publication Ltd., London, GB; AN 75-20002 & JP-A 49097793 (Asahi Chemical Ind. Co.) 19. September 1974 empfiehlt die thermische Behandlung entsprechender inniger Trockengemische zur Herstellung relevanter Multielementoxidaktivmassen unter völligem Ausschluss von molekularem Sauerstoff. Die EP-A 113 156 empfiehlt, die thermische Behandlung im Luftstrom durchzuführen. Die EP-A 724481 lehrt, die thermische Behandlung so durchzuführen, dass der Gehalt an molekularem Sauerstoff zu jedem Zeitpunkt der thermischen Behandlung in der (gasförmigen) Behandlungsatmosphäre 0,5 bis 4 Vol.-% beträgt. In der beispielhaften Ausführungsform betrug der molekulare Sauerstoffgehalt in der thermischen Behandlungsatmosphäre 1,5 Vol.-%.

In den beispielhaften Ausführungsformen der EP-A 714700 wird die thermische Behandlung des innigen Trockengemischs sowohl im Luftstrom als auch in einer Atmosphäre durchgeführt, deren molekularer Sauerstoffgehalt 1,5 Vol.% betrug.

Die DE-A 10046928, die DE-A 19815281 und die EP-A 668104 weisen aus, dass eingangs erwähnte Multielementoxidaktivmassen, die eine mehrphasige Struktur aufweisen, dann als Aktivmassen für Katalysatoren zur heterogen katalysierten Partialoxidation von Acrolein zu Acrylsäure besonders geeignet sind, wenn man zur Herstellung des thermisch zu behandelnden innigen Trockengemisches wenigstens eine Phase getrennt vorbildet und die thermische Behandlung in einer Gasatmosphäre durchführt, die stetig 1,5 bzw. 1,4 Vol.-% an molekularem Sauerstoff enthält.

Nachteilig an den Lehren des Standes der Technik ist, dass sie im wesentlichen alle eine thermische Behandlung des innigen Trockengemischs bei einem über die Zeitdauer der thermischen Behandlung im wesentlichen konstanten Gehalt an molekularem Sauerstoff in der zugehörigen Gasatmosphäre empfehlen.

Solchermaßen erhaltene relevante Multielementoxidaktivmassen vermögen jedoch bezüglich Aktivität und Selektivität bei Verwendung als Aktivmassen in Katalysatoren für die heterogen katalysierte Partialoxidation von Acrolein zu Acrylsäure nicht voll zu befriedigen.

Die Anmeldung EP20040739464 betrifft ein Verfahren zur thermischen Behandlung von Vorläufermassen im Drehrohrofen, die jenen in der Präambel dieser Anmeldung ähnlich sind, und bei dem das Zeitmittel der Gasatmosphäre, in der die thermische Behandlung erfolgt, ein spezifisches ist.

Die Aufgabe der vorliegenden Erfindung bestand daher darin, ein verbessertes Verfahren zur Herstellung relevanter Multielementoxidaktivmassen zur Verfügung zu stellen, gemäß dessen Multielementoxidaktivmassen erhalten werden, die bei Verwendung als Aktivmassen in Katalysatoren für die heterogen katalysierte Partialoxidation von Acrolein zu Acrylsäure eine erhöhte Aktivität und eine erhöhte Selektivität der Acrylsäurebildung aufweisen.

Demgemäss wurde ein Verfahren zur Herstellung von katalytisch aktiven Multielementoxidmassen, die wenigstens eines der Elemente Nb und W sowie die Elemente Mo, V und Cu enthalten, wobei der molare Anteil des Elements Mo an der Gesamtmenge aller von Sauerstoff verschiedenen Elemente der katalytisch aktiven Multielementoxidmasse 20 mol-% (vorzugsweise 30 mol% oder 40 mol-%) bis 80 mol% beträgt, das molare Verhältnis von in der katalytisch aktiven Multielementoxidmasse enthaltenem Mo zu in der katalytisch aktiven Multielementoxidmasse enthaltenem V, Mo/V, 15:1 bis 1:1 beträgt, das entsprechende molare Verhältnis Mo/Cu 30:1 bis 1:3 und das entsprechende molare Verhältnis Mo/(Gesamtmenge aus W und Nb) 80:1 bis 1:4 beträgt und bei dem man Ausgangsverbindungen, die die von Sauerstoff verschiedenen elementaren Konstituenten der Multielementoxidmasse als Bestandteile enthalten, ein inniges, auch Ammoniumionen enthaltendes, Trockengemisch herstellt und dieses in einer an molekularem Sauerstoff armen (Gas) Atmosphäre bei erhöhter Temperatur thermisch behandelt, wobei bei Temperaturen ≥160°C wenigstens eine Teilmenge der im innigen Trockengemisch enthaltenen Ammoniumionen unter Freisetzung von Ammoniak zersetzt wird, gefunden, das dadurch gekennzeichnet ist, dass die thermische Behandlung wie folgt erfolgt:
- das innige Trockengemisch wird mit einer Temperaturrate von ≥10°C/min auf eine Zersetzungstemperatur im Zersetzungstemperaturbereich von 240°C bis 360°C erwärmt und so lange in diesem Temperaturbereich gehalten, bis wenigstens 90 mol-% der im Gesamtverlauf der thermischen Behandlung des innigen Trockengemischs aus dem innigen Trockengemisch bei Temperaturen oberhalb von 160°C insgesamt freigesetzten Gesamtmenge M^{A} an Ammoniak freigesetzt worden sind;
- spätestens dann, wenn das innige Trockengemisch die Temperatur von 230°C erreicht hat, wird der Gehalt der (Gas) Atmosphäre A, in der sich die thermische Behandlung des innigen Trockengemisches vollzieht, an molekularem Sauerstoff auf einen Wert von ≤0,5 Vol.-% abgesenkt und dieser niedere Sauerstoffgehalt so lange aufrechterhalten, bis wenigstens 20 mol-%, bevorzugt wenigstens 30 mol-% und besonders bevorzugt wenigstens 40 mol-% der im Gesamtverlauf der thermischen Behandlung insgesamt freigesetzten Gesamtmenge M^{A} an Ammoniak freigesetzt worden sind;
- frühestens dann, wenn ≥ 70 mol-% (häufig wenn ≥75 mol-%, oder wenn ≥ 80 mol%, oder wenn ≥85 mol%, oder wenn ≥90 mol-%) der im Gesamtverlauf der thermischen Behandlung insgesamt freigesetzten Gesamtmenge M^{A} an Ammoniak freigesetzt worden sind, wird das innige Trockengemisch mit einer Rate von ≥10°C aus dem Zersetzungstemperaturbereich heraus - und in den Calcinationstemperaturbereich von 380 bis 450°C hineingeführt und
- spätestens dann, wenn 98 mol-% bzw. 95 mol-% der im Gesamtverlauf der thermischen Behandlung insgesamt freigesetzten Gesamtmenge M^{A} an Ammoniak freigesetzt worden sind, wird der Gehalt der Atmosphäre A an molekularem Sauerstoff auf > 0,5 Vol.% bis 4 Vol.% erhöht
und das innige Trockengemisch bei diesem erhöhten Sauerstoffgehalt der Atmosphäre A im Calcinationstemperaturbereich calciniert.

Selbstredend sind beim erfindungsgemäßen Verfahren die Elemente Mo, V, Cu sowie Nb und/oder W (ebenso wie alle anderen gegebenenfalls enthaltenen und von Sauerstoff verschiedenen Elemente) in der erfindungsgemäß erhältlichen katalytisch aktiven Multielementoxidmasse in oxidischer (und nicht in metallischer, elementarer) Form enthalten.

Der Gehalt des erfindungsgemäß thermisch zu behandelnden innigen Trockengemischs an Ammoniumionen beträgt mit Vorteil, bezogen auf den molaren Gesamtgehalt des innigen Trockengemischs an von Sauerstoff verschiedenen elementaren Konstituenten der späteren katalytisch aktiven Multielementoxidmasse, wenigstens 5 oder wenigstens 10 mol-%, vorzugsweise wenigstens 20 mol-%, besonders bevorzugt wenigstens 30 mol% und ganz besonders bevorzugt wenigstens 40 mol%. In der Regel beträgt der so bezogene Ammoniumgehalt des innigen Trockengemischs ≤150 mol-% bzw. ≤100 mol-%, meist ≤90 mol-% oder ≤80 mol%, häufig ≤70 mol-% oder ≤60 mol-%.

Die Temperaturrate, mit der das innige Trockengemisch auf die Zersetzungstemperatur erwärmt wird, beträgt beim erfindungsgemäßen Verfahren mit Vorteil ≤8°C/min, bevorzugt ≤5°C/min, besonders bevorzugt ≤3°C/min, ganz besonders bevorzugt ≤2°C/min oder ≤1°C/min. In der Regel wird diese Temperaturrate jedoch ≥0,1 °C/min, meist ≥0,2°C/min und häufig ≥0,3°C/min oder ≥0,4°C/min betragen.

Das Vorgenannte gilt auch für die Temperaturrate mit der das innige Trockengemisch aus dem Zersetzungsbereich heraus - und in den Calcinationsbereich von 380 bis 480°C hineingeführt wird.

Erfindungsgemäß bevorzugt erstreckt sich der Zersetzungstemperaturbereich auf den Bereich von 280 bis 360°C und besonders bevorzugt auf den Bereich von 300 bis 350°C bzw. 310 bis 340°C.

Ferner wird das innige Trockengemisch beim erfindungsgemäßen Verfahren mit Vorteil so lange im Zersetzungstemperaturbereich gehalten, bis wenigstens 95 mol%, besser bis wenigstens 97 mol-% und noch besser bis wenigstens 99 mol% oder die Gesamtmenge der im Gesamtverlauf der thermischen Behandlung des innigen Trockengemischs insgesamt freigesetzten Gesamtmenge M^{A} an Ammoniak freigesetzt worden sind.

Normalerweise wird beim erfindungsgemäßen Verfahren das innige Trockengemisch von Raumtemperatur (das sind z.B. 20°C, oder 25°C, oder 30°C, oder 35°C, oder 40°C) ausgehend auf die Zersetzungstemperatur erwärmt.

Spätestens dann, wenn das innige Trockengemisch die Temperatur von 230°C erreicht hat, muss beim erfindungsgemäßen Verfahren der Gehalt der (Gas) Atmosphäre A, in der sich die thermische Behandlung des innigen Trockengemischs vollzieht, an molekularem Sauerstoff auf einen Wert von ≤0,5 Vol.% abgesenkt werden.

Erfindungsgemäß bevorzugt erfolgt diese Absenkung des Gehalts an molekularem Sauerstoff auf einen Wert von ≤0,3 Vol.-% und besonders bevorzugt auf einen Wert von ≤0,1 Vol.-%. Besonders bevorzugt ist in dieser Phase des erfindungsgemäßen Verfahrens der Gehalt der Atmosphäre A an molekularem Sauerstoff verschwindend. In der Regel wird dieser Sauerstoffgehalt jedoch bei Werten von ≥0,05 Vol.% liegen.

Bevorzugt liegt der Gehalt der Atmosphäre A beim erfindungsgemäßen Verfahren auch bereits unterhalb der Temperatur von 230°C (z.B. schon bei Temperaturen ≥200°C) bei ≤0,5 Vol.-%, bzw. ≤0,3 Vol.%, bzw. ≤0,1 Vol.-% oder bei 0 Vol.-%. In der Regel wird dieser Sauerstoffgehalt jedoch auch unterhalb von 230°C (z.B. auch bei Temperaturen < 200°C) bei Werten von ≥0,05 Vol.% liegen.

Unterhalb der 230°C bzw. 200°C kann die Gasatmosphäre A, in der sich die thermische Behandlung vollzieht, aber auch deutlich höhere Sauerstoffgehalte aufweisen. Prinzipiell kann dieser Gehalt an molekularem Sauerstoff unterhalb der 230°C bzw. 200°C beim erfindungsgemäßen Verfahren ≥5 Vol.-%, oder ≥10 Vol.-%, oder ≥15 Vol.%, oder ≥20 Vol.-%, oder ≥25 Vol.-%, oder 30 Vol.-%, oder mehr betragen. Auch eine im wesentlichen ausschließlich aus Luft oder aus molekularem Sauerstoff bestehende thermische Behandlungsatmosphäre ist in diesem Temperaturbereich möglich.

Erfindungsgemäß wird der Gehalt der Atmosphäre A an molekularem Sauerstoff von ≤0,5 Vol.-% wenigstens so lange aufrechterhalten, bis wenigstens 20 mol%, oder 30 mol%, oder 40 mol%, bevorzugt bis wenigstens 50 mol-%, besonders bevorzugt bis wenigstens 60 mol-%, oder wenigstens 70 mol-%, oder wenigstens 80 mol% der im Gesamtverlauf der thermischen Behandlung insgesamt freigesetzten Gesamtmenge M^{A} an Ammoniak freigesetzt worden sind.

Erfahrungsgemäß vorteilhaft wird man den Gehalt der Atmosphäre A an molekularem Sauerstoff jedoch bereits dann auf einen Wert > 0,5 Vol.% bis 4 Von.-% erhöhen, bevor 95 mol% der im Gesamtverlauf der thermischen Behandlung insgesamt freigesetzten Gesamtmenge M^{A} an Ammoniak freigesetzt worden sind. Bevorzugt erfolgt diese Erhöhung des Sauerstoffgehalts bereits bevor 90 mol-%, vorzugsweise bevor 85 mol-% und besonders bevorzugt spätestens dann wenn 80 mol% der im Gesamtverlauf der thermischen Behandlung insgesamt freigesetzten Gesamtmenge M^{A} an Ammoniak freigesetzt worden sind.

D.h. erfindungsgemäß zweckmäßig erstreckt sich der Bereich des erfindungsgemäßen Verfahrens, in dem der Gehalt der Atmosphäre A an molekularem Sauerstoff ≤0,5 Vol.% beträgt, soweit, bis 20, bzw. 30, bzw. 40 bis 80 mol-% und bevorzugt bis 50 bis 70 mol% der im Gesamtverlauf der thermischen Behandlung insgesamt freigesetzten Gesamtmenge M^{A} an Ammoniak freigesetzt worden sind.

Wird der Gehalt der Atmosphäre A an molekularem Sauerstoff spätestens dann, wenn 98 mol-% bzw. 95 mol-% der im Gesamtverlauf der thermischen Behandlung insgesamt freigesetzten Gesamtmenge M^{A} an Ammoniak freigesetzt worden sind, auf einen Wert ≥0,5 Vol.-% bis 4 Vol.-% erhöht, so erfolgt diese Erhöhung bevorzugt auf einen ≥0,55 Vol.-% bis 4 Vol.-%, und besonders bevorzugt auf einen ≥0,6 Vol.-% bis 4 Vol.-% betragenden Wert. Ganz besonders bevorzugt erfolgt die Erhöhung des Sauerstoffgehalts auf einen Wert von 1 bis 3 Vol.% bzw. von 1 bis 2 Vol.%. Diese Erhöhungswerte gelten auch für Erhöhungen bei allen anderen als möglich genannten freigesetzten Teilmengen der insgesamt freigesetzten Gesamtmengen an Ammoniak.

Der Calcinationstemperaturbereich erstreckt sich beim erfindungsgemäßen Verfahren mit Vorteil auf eine Temperatur des innigen Trockengemischs von 380 bis 430°C und besonders bevorzugt auf eine solche Temperatur von 390 bis 420°C.

Der Zersetzungstemperaturbereich ist beim erfindungsgemäßen Verfahren derjenige Temperaturbereich, nach dessen Durchlaufen die Zersetzung der im erfindungsgemäß thermisch zu behandelnden innigen Trockengemisch enthaltenen Ammoniumionen weitgehend abgeschlossen ist.

Im Calcinationstemperaturbereich erfolgt beim erfindungsgemäßen Verfahren die Ausbildung des katalytisch aktiven Multielementoxids.

In der Regel wird die Calcination im Calcinationstemperaturbereich wenigstens 10 min, vorzugsweise wenigstens 20 min und besonders bevorzugt wenigstens 30 min andauern. In der Regel erstreckt sich die Calcination im Calcinationstemperaturbereich auf ≤2 h, häufig ≤1,5 h bzw. ≤1 h.

Nach beendeter Calcination wird das Calcinationsgut normalerweise abgekühlt. In der Regel erfolgt diese Abkühlung auf Raumtemperatur (d.h., z.B. auf 20°C, oder auf 25°C, oder auf 30°C, oder auf 35°C, oder auf 40°C).

Erfindungsgemäß zweckmäßig vollzieht sich die Abkühlung des Calcinationsguts auf eine ≤100°C betragende Temperatur innerhalb eines Zeitraums von ≤5 h, bevorzugt ≤4 h, besonders bevorzugt ≤3 h oder ≤2 h. In der Regel wird dieser Abkühlzeitraum aber nicht weniger als 0,5 h betragen.

Mit Vorteil erfolgt die Abkühlung des Calcinationsguts in einer es umgebenden (Gas) Atmopshäre A, deren Gehalt an molekularem Sauerstoff ≤5 Vol.%, oder ≤4 Vol.%, oder ≤3 Vol.-%, oder ≤2 Vol.%, oder ≤1 Vol.%, oder ≤0,5 Vol.%, bevorzugt ≤0,3 Vol.% bzw. ≤0,1 Vol.% oder 0 Vol.%, in der Regel ≤0,05 Vol.% beträgt. Dieser Sauerstoffgehalt wird zweckmäßig dann eingestellt, wenn noch im Calcinationstemperaturbereich befindlich damit begonnen wird, das Calcinationsgut durch Temperaturerniedrigung aus dem Calcinationstemperaturbereich herauszuführen.

Ist das Calcinationsgut auf eine Temperatur von ≤350°C bzw. ≤300°C bzw. ≤250°C, abgekühlt, kann die weitere Abkühlung auch in einer (Gas) Atmosphäre A erfolgen, deren Gehalt an molekularem Sauerstoff > 5 Vol.%, oder ≥10 Vol.%, oder ≥15 Vol.-%, oder ≥20 Vol.-%, oder ≥25 Vol.%, oder ≥30 Vol.-%, oder mehr beträgt.

Auch eine im wesentlichen ausschließlich aus Luft oder aus molekularem Sauerstoff bestehende (Gas) Atmosphäre A ist beim weiteren Abkühlen unter diese Temperatur möglich.

Neben den beschriebenen Gehalten an molekularem Sauerstoff wird sich die Atmosphäre A, in der sich die thermische Behandlung des innigen Trockengemischs vollzieht, im wesentlichen aus den aus dem innigen Trockengemisch gasförmig entweichenden Bestandteilen und aus Inertgas zusammensetzen. Unter dem Begriff "Inertgase" werden dabei all jene Gase verstanden, die auf die erfindungsgemäß thermisch zu behandelnde Trockenmasse nicht chemisch reaktiv einwirken. Beispiele für Inertgase sind N₂ oder Edelgase. Wasserdampf wird die Atmosphäre A insbesondere dann aufweisen, wenn das innige Trockengemisch Hydratwasser enthält. In der RegeII wird der Wasserdampfanteil der Atmosphäre A zu keinem Zeitpunkt während der erfindungsgemäßen thermischen Behandlung 20 Vol.-% überschreiten. In der Regel wird er sogar zu allen Zeitpunkten ≤10 Vol.% betragen.

Der Ammoniakgehalt der (Gas) Atmosphäre A wird beim erfindungsgemäßen Verfahren normalerweise ein Maximum durchlaufen, das normalerweise ≤10 Vol.-%, häufig ≤8 Vol.% und meist ≤7 Vol.-% beträgt. Üblicherweise wird es jedoch oberhalb von 1 Vol.%, häufig oberhalb von 2 Vol.% oder 3 Vol.% liegen.

Normalerweise wird der Ammoniakgehalt der Atmosphäre A sein Maximum durchlaufen, bevor das innige Trockengemisch den Calcinationstemperaturbereich erreicht hat.

D.h. im Calcinationstemperaturbereich wird der maximale Ammoniakgehalt der Atmosphäre A in der Regel bei Werten ≤2 Vol.%, bzw. ≤1 Vol.%, liegen. Er wird jedoch üblicherweise bei Werten > 0 Vol.% liegen.

Über die Gesamtdauer des Aufenthalts des innigen Trockengemisch im Calcinationstemperaturbereich gemittelt (arithmetisch) wird der NH₃-Gehalt der Atmosphäre A in der Regel ≤1 Vol.%, bevorzugt ≤0,5 Vol.% betragen. Über die Gesamtdauer des Aufenthalts des innigen Trockengemischs im Temperaturbereich > 160°C, ≤360°C gemittelt (arithmetisch) wird der NH₃-Gehalt der Atmosphäre A normalerweise 1 bzw. 1,5 oder 2 bis 8 Vol.%, meist 1 bis 4 Vol.-% betragen.

Üblicherweise wird beim erfindungsgemäßen Verfahren der (Gas) Atmosphäre A kein externer Ammoniak zugeführt. D.h., die einzige Ammoniakquelle sind normalerweise die ins innige Trockengemisch eingearbeiteten Ammoniumionen. Allerdings kann es beim erfindungsgemäßen Verfahren zweckmäßig sein, kontinuierlich (Gas) Atmosphäre A zu entnehmen und in gewissem Umfang im Kreis zu fahren (d.h. zum thermisch zu behandelnden innigen Trockengemisch rückzuführen).

Erfindungsgemäß vorteilhaft wird die Calcination abgebrochen, bevor im Röntgendiffraktogramm MoO₃ nachweisbar ist. Jedoch sind in den erfindungsgemäß erhältlichen Multielementoxidaktivmassen MoO₃-Gehalte von bis zu 30 Gew.-% bzw. bis zu 20 Gew.-% tolerabel.

Neben den Elementen Nb und/oder W, sowie Mo, V und Cu können die erfindungsgemäß erhältlichen Multielementoxidaktivmassen zusätzlich z.B. die Elemente Ta, Cr, Ce, Ni, Co, Fe, Mn, Zn, Sb, Bi, Alkali (Li, Na, K, Rb, Cs), H, Erdalkali (Mg, Ca, Sr, Ba), Si, Al, Ti und Zr enthalten. Natürlich kann die Multielementoxidaktivmasse erfindungsgemäß aber auch nur aus den Elementen Nb und/oder W sowie Mo, V und Cu bestehen.

Als Aktivmasse für Katalysatoren zur heterogen katalysierten partiellen Gasphasenoxidation von Acrolein zu Acrylsäure (sowie von Methacrolein zu Methacrylsäure sowie von Propan zu Acrylsäure; für diese heterogen katalysierten Gasphasenpartialoxidationen eignen sich die erfindungsgemäßen Verfahrensprodukte ebenfalls) besonders geeignete erfindungsgemäß erhältliche katalytisch aktive Multielementoxidmassen genügen der nachfolgenden allgemeinen Stöchiometrie I

Mo₁₂VₐX¹_{b}X²_{c}X³_{d}X⁴ₑX⁵_{f}X⁶_{g}Oₙ (I),

in der die Variablen folgende Bedeutung haben:
- X¹: = W, Nb, Ta, Cr und/oder Ce,
- X²: = Cu, Ni, Co, Fe, Mn und/oder Zn,
- X³: = Sb und/oder Bi,
- X⁴: = einer oder mehrere Alkalimetalle (Li, Na, K, Rb, Cs) und/oder H,
- X⁵: = eines oder mehrere Erdalkalimetalle (Mg, Ca, Sr, Ba),
- X⁶: = Si, Al, Ti und/oder Zr,
- a: = 1 bis 6,
- b: = 0,2 bis 4,
- c: = 0,5 bis 18,
- d: = 0 bis 40,
- e: = 0 bis 2,
- f: = 0 bis 4,
- g: = 0 bis 40 und
- n: = eine Zahl, die durch die Wertigkeit und Häufigkeit der von Sauerstoff verschiedenen Elemente in I bestimmt wird, und
wobei die Variablen innerhalb der vorgegebenen Bereiche mit der Maßgabe auszuwählen sind, dass der molare Anteil des Elementes Mo an der Gesamtmenge aller von Sauerstoff verschiedenen Elemente der Multielementoxidmasse (I) 20 mol-% bis 80 mol-% beträgt, das molare Verhältnis von in der katalytisch aktiven Multielementoxidmasse (I) enthaltenem Mo zu in der katalytisch aktiven Multielementoxidmasse (I) enthaltenem V, Mo/V, 15:1 bis 1:1 beträgt, das entsprechende molare Verhältnis Mo/Cu 30:1 bis 1:3 und das entsprechende molare Verhältnis Mo/(Gesamtmenge aus W und Nb) 80:1 bis 1:4 beträgt.

Unter den aktiven Multielementoxidmassen (I) sind jene bevorzugt, bei denen die Variablen in den folgenden Bereichen liegen:
- x¹: = W, Nb und/oder Cr,
- X²: = Cu, Ni, Co und/oder Fe,
- X³: = Sb, ,
- X⁴: = Na und/oder K,
- X⁵: = Ca, Sr und/oder Ba,
- X⁶: = Si, Al und/oder Ti,
- a: = 2,5 bis 5,
- b: = 0,5 bis 2,
- c: = 0,5 bis 3,
- d: = 0 bis 2,
- e: = 0 bis 0,2,
- f: = 0 bis 1,
- g: = 0 bis 15 und
- n: = eine Zahl, die durch die Wertigkeit und Häufigkeit der von Sauerstoff verschiedenen Elemente in I bestimmt wird.

Ganz besonders bevorzugt sind jedoch die nachfolgenden Multielementoxidaktivmassen II unmittelbare Verfahrensprodukte des erfindungsgemäßen Verfahrens:

Mo₁₂VₐX¹_{b}X²_{c}X⁵_{f}X⁶_{g}Oₙ (II),

in der die Variablen folgende Bedeutung haben:
- X¹: = W und/oder Nb,
- X²: = Cu und/oder Ni,
- X⁵: = Co und/oder Sr,
- X⁶: = Si und/oder Al,
- a: = 3 bis 4,5,
- b: = 1 bis 1,5,
- c: = 0,75 bis 2,5,
- f: = 0 bis 0,5,
- g: = 0 bis 8 und
- n: = eine Zahl, die durch die Wertigkeit und Häufigkeit der von Sauerstoff verschiedenen Elemente in II bestimmt wird, und
wobei die Variablen innerhalb der vorgegebenen Bereiche mit der Maßgabe auszuwählen sind, dass der molare Anteil des Elementes Mo an der Gesamtmenge aller von Sauerstoff verschiedenen Elemente der Multielementoxidaktivmasse (II) 20 mol% bis 80 mol% beträgt, das molare Verhältnis von in der katalytisch aktiven Multielementoxidmasse (II) enthaltenem Mo zu in der katalytisch aktiven Multielementoxidmasse (II) enthaltenem V, Mo/V, 15:1 bis 1:1 beträgt, das entsprechende molare Verhältnis Mo/Cu 30:1 bis 1:3 und das entsprechende molare Verhältnis Mo/(Gesamtmenge aus W und Nb) 80:1 bis 1:4 beträgt.

Zur Herstellung von solchen erfindungsgemäßen unmittelbaren Verfahrensprodukten geht man beim erfindungsgemäßen Verfahren von in an sich bekannter Weise geeigneten Quellen (Ausgangsverbindungen) der von Sauerstoff verschiedenen elementaren Konstituenten der gewünschten Multielementoxidaktivmasse im in der Multielementoxidaktivmasse angestrebten jeweiligen stöchiometrischen Verhältnis aus, und erzeugt aus diesen ein möglichst inniges, vorzugsweise feinteiliges, Trockengemisch, welches dann der erfindungsgemäßen thermischen Behandlung unterworfen wird, wobei die thermische Behandlung vor oder nach der Formung zu Katalysatorformkörpern bestimmter Geometrie erfolgen kann. Erfindungsgemäß vorteilhaft erfolgt sie davor. Dabei kann es sich bei den Quellen entweder bereits um Oxide handeln, oder um solche Verbindungen, die durch Erhitzen, wenigstens in Anwesenheit von Sauerstoff, in Oxide überführbar sind. Neben den Oxiden kommen daher als Ausgangsverbindungen vor allem Halogenide, Nitrate, Formiate, Oxalate, Acetate, Carbonate oder Hydroxide in Betracht.

Geeignete Ausgangsverbindungen des Mo, V, W und Nb sind auch deren Oxoverbindungen (Molybdate, Vanadate, Wolframate und Niobate) bzw. die von diesen abgeleiteten Säuren. Sauerstoff haltige Quellen sind für das erfindungsgemäße Verfahren günstig.

Der erfindungsgemäß erforderliche Gehalt des innigen Trockengemischs an Ammoniumionen kann in einfacher Weise dadurch realisiert werden, dass man in das innige Trockengemisch eine entsprechende Menge Ammoniumionen einarbeitet. Zweckmäßigerweise lassen sich die Ammoniumionen in das innige Trockengemisch z.B. dadurch einbringen, dass man als Quellen der Elemente Mo, V, W oder Nb die entsprechenden Ammoniumoxometallate verwendet. Beispiele hierfür sind Ammoniummetaniobat, Ammoniummetavanadat, Ammoniumheptamolybdattetrahydrat und Ammoniumparawolframatheptahydrat. Selbstverständlich können in das thermisch zu behandelnde innige Trockengemisch aber auch unabhängig von den als Quellen der Multielementoxidaktivmassenkonstituenten erforderlichen Ausgangsverbindungen Ammoniumlieferanten wie NH₄NO₃, oder NH₄Cl, oder Ammoniumacetat, oder Ammoniumcarbonat, oder Ammoniumhydrogencarbonat, oder NH₄OH, oder NH₄CHO₂, oder Ammoniumoxalat eingearbeitet werden.

Das innige Vermischen der Ausgangsverbindungen kann prinzipiell in trockener oder in nasser Form erfolgen. Erfolgt es in trockener Form, werden die Ausgangsverbindungen zweckmäßigerweise als feinteilige Pulver eingesetzt und nach dem Mischen z.B. zu Katalysatorformkörpern gewünschter Geometrie verpresst (z.B. tablettiert), die dann der erfindungsgemäßen thermischen Behandlung unterworfen werden.

Vorzugsweise erfolgt das innige Vermischen jedoch in nasser Form. Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen und Ausgangsverbindungen ausgegangen wird. Als Lösungsmittel wird bevorzugt Wasser eingesetzt. Anschließend wird die wässrige Masse (Lösung oder Suspension) getrocknet und das so erhaltene innige Trockengemisch gegebenenfalls unmittelbar erfindungsgemäß thermisch behandelt. Vorzugsweise erfolgt der Trocknungsprozess durch Sprühtrocknung (die Austrittstemperaturen betragen in der Regel 100 bis 150°C) und unmittelbar im Anschluss an die Fertigstellung der wässrigen Lösung oder Suspension. Das dabei anfallende Pulver kann unmittelbar durch Pressen geformt werden. Häufig erweist es sich jedoch für eine unmittelbare Weiterverarbeitung als zu feinteilig, weshalb es dann unter Zusatz von z.B. Wasser zweckmäßigerweise geknetet wird. Vielfach erweist sich beim Kneten ein Zusatz einer niederen organischen Carbonsäure (z.B. Essigsäure) als vorteilhaft (typische Zusatzmengen liegen bei 5 bis 10 Gew.-% bezogen auf eingesetzte Pulvermasse).

Die anfallende Knetmasse wird anschließend entweder zur gewünschten Katalysatorgeometrie geformt, getrocknet und dann der erfindungsgemäßen thermischen Behandlung unterworfen (führt zu sogenannten Vollkatalysatoren) oder ungeformt calciniert und danach zu einem Pulver vermahlen (üblicherweise < 80 µm, vorzugsweise < 50 µm, besonders bevorzugt < 30 µm, in der Regel ≥1 µm) welches normalerweise unter Zusatz einer geringen Menge Wasser sowie gegebenenfalls weiterer üblicher Bindemittel als Feuchtmasse auf inerte Trägerkörper aufgetragen wird. Nach beendeter Beschichtung wird nochmals getrocknet und so ein einsatzfähiger Schalenkatalysator erhalten. Erfolgt das innige Vermischen der Ausgangsverbindungen in Form einer z.B. wässrigen Lösung, so können mit selbiger auch inerte poröse Trägerkörper getränkt, getrocknet und anschließend zu Trägerkatalysatoren erfindungsgemäß thermisch behandelt werden. Bei der Herstellung von Schalenkatalysatoren kann das Beschichten der Trägerkörper auch vorab der erfindungsgemäßen thermischen Behandlung, d.h. z.B. mit dem befeuchteten Sprühpulver, erfolgen.

Für Schalenkatalysatoren geeignete Trägermaterialien sind z.B. poröse oder unporöse Aluminiumoxide, Siliciumdioxid, Thoriumdioxid, Zirkondioxid, Siliciumcarbid oder Silikate wie Magnesium- oder Aluminiumsilicat (z.B. Steatit des Typs C 220 der Fa. CeramTec).

Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder mit Splittauflage, bevorzugt werden.

Geeignet ist die Verwendung von im wesentlichen unporösen, oberflächenrauhen, kugelförmigen Trägern aus Steatit (z.B. Steatit des Typs C 220 der Fa. CeramTec), deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Bevorzugt zu verwendende ringförmige Trägerkörper besitzen eine Länge von 2 bis 6 mm, einen Außendurchmesser von 4 bis 8 mm und eine Wanddicke von 1 bis 2 mm. Geeignet sind vor allem auch Ringe der Geometrie 7 mm x 3 mm x 4 mm (Außendurchmesser x Länge x Innendurchmesser) als Trägerkörper.

Die Beschichtung der Trägerkörper mit feinteiliger erfindungsgemäß erhältlicher Multielementoxidaktivmasse bzw. deren erfindungsgemäß thermisch zu behandelnder feinteiliger Vorlaufsmasse (innigesTrockengemisch) wird in der Regel in einem drehbaren Behälter ausgeführt, wie es z.B. aus der DE-A 2909671, der EP-A 293859 oder aus der EP-A 714700 bekannt ist. Die Verfahrensweise der EP-A 714700 ist dabei bevorzugt.

Zweckmäßigerweise wird zur Beschichtung der Trägerkörper mit der aufzubringenden Pulvermasse der Trägerkörper befeuchtet. Nach dem Aufbringen wird normalerweise mittels heißer Luft getrocknet. Die Schichtdicke der auf den Träger aufgebrachten Pulvermasse wird zweckmäßigerweise im Bereich 10 bis 1000 µm, bevorzugt im Bereich 50 bis 500 µm und besonders bevorzugt im Bereich 150 bis 250 µm liegend, gewählt.

Im Fall von Vollkatalysatoren kann die Formgebung, wie bereits erwähnt, ebenfalls vor oder nach der Durchführung der erfindungsgemäßen thermischen Behandlung erfolgen.

Beispielsweise können aus der Pulverform der erfindungsgemäß erhältlichen Multielementoxidaktivmasse oder ihrer noch nicht thermisch behandelten Vorläufermasse (dem innigen Trockengemisch) durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Selbstverständlich können die erfindungsgemäß erhältlichen Multielementoxidaktivmassen auch in Pulverform, d.h. nicht zu bestimmten Katalysatorgeometrien geformt, als Katalysatoren für die heterogen katalysierte Partialoxidation von Acrolein zu Acrylsäure, bzw. Methacrolein zu Methacrylsäure bzw. Propan zu Acrylsäure eingesetzt werden (z.B. auch im Wirbelbett).

Das erfindungsgemäße Verfahren eignet sich aber auch zur Herstellung von Multielementoxidaktivmassen der allgemeinen Formel III,

[A]ₚ[B]_{q}[C]ᵣ (III),

in der die Variablen folgende Bedeutung haben:
- A -: Mo₁₂VₐX¹_{b}X²_{c}X³_{d}X⁴ₑX⁵_{f}X⁶_{g}Oₓ,
- B: = X₁⁷CuₕHᵢO_{y},
- C =: X₁⁸SbⱼHₖO_{z},
- X¹: = W, Nb, Ta, Cr und/oder Ce, vorzugsweise W, Nb und/oder Cr,
- X²: = Cu, Ni, Co, Fe, Mn und/oder Zn, vorzugsweise Cu, Ni, Co und/oder Fe,
- X³: = Sb und/oder Bi, vorzugsweise Sb,
- X⁴: = Li, Na, K, Rb, Cs und/oder H. vorzugsweise Na und/oder K,
- X⁵: = Mg, Ca, Sr und/oder Ba, vorzugsweise Ca, Sr und/oder Ba,
- X⁶: = Si, Al, Ti und/oder Zr, vorzugsweise Si, Al und/oder Ti,
- X⁷: = Mo, W, V, Nb und/oder Ta, vorzugsweise Mo und/oder W,
- X⁸: = Cu, Ni, Zn, Co, Fe, Cd, Mn, Mg, Ca, Sr und/oder Ba, vorzugsweise Cu und/oder Zn, besonders bevorzugt Cu,

- a: = 1 bis 8, vorzugsweise 2 bis 6,
- b: = 0,2 bis 5, vorzugsweise 0,5 bis 2,5,
- c: = 0 bis 23, vorzugsweise 0 bis 4,
- d: = 0 bis 50, vorzugsweise 0 bis 3,
- e: = 0 bis 2, vorzugsweise 0 bis 0,3,
- f: = 0 bis 5, vorzugsweise 0 bis 2,
- g: = 0 bis 50, vorzugsweise 0 bis 20,
- h: = 0,3 bis 2,5, vorzugsweise 0,5 bis 2, besonders bevorzugt 0,75 bis 1,5,
- i: = 0 bis 2, vorzugsweise 0 bis 1,
- j: = 0,1 bis 50, vorzugsweise 0,2 bis 20, besonders bevorzugt 0,2 bis 5,
- k: = 0 bis 50, vorzugsweise 0 bis 20, besonders bevorzugt 0 bis 12,
- x,y,z: = Zahlen, die durch die Wertigkeit und Häufigkeit der von Sauerstoff verschiedenen Elementen in A, B, C bestimmt werden,
- p, q =: positive Zahlen,
- r: = 0 oder eine positive Zahl, vorzugsweise eine positive Zahl, wobei das Verhältnis p/(q+r) = 20:1 bis 1:20, vorzugsweise 5:1 bis 1:14 und besonders bevorzugt 2:1 bis 1:8 und , für den Fall, dass r eine positive Zahl ist, das Verhältnis q/r = 20:1 bis 1:20, vorzugsweise 4:1 bis 1:4, besonders bevorzugt 2:1 bis 1:2 und ganz besonders bevorzugt 1:1 beträgt,
die den Anteil [A]p in Form dreidimensional ausgedehnter Bereiche (Phasen) A der chemischen Zusammensetzung
A: Mo₁₂VaX¹_{b}X²_{c}X³_{d}X⁴ₑX⁵_{f}X⁶_{g}Oₓ,
den Anteil [B]q in Form dreidimensional ausgedehnter Bereiche (Phasen) B der chemischen Zusammensetzung
B : X₁⁷CuₕHᵢO_{y} und
den Anteil [C]ᵣ in Form dreidimensional ausgedehnter Bereiche (Phasen) C der chemischen Zusammensetzung
C : X₁⁸SbⱼHₖO_{z}
enthalten, wobei die Bereiche A, B und gegebenenfalls C relativ zueinander wie in einem Gemisch aus feinteiligem A, feinteiligem B und gegebenenfalls feinteiligem C verteilt sind, und
wobei alle Variablen innerhalb der vorgegebenen Bereiche mit der Maßgabe auszuwählen sind, dass der molare Anteil des Elements Mo an der Gesamtmenge aller von Sauerstoff verschiedenen Elemente der Multielementoxidaktivmasse (III) 20-mol% bis 80 mol% beträgt, das molare Verhältnis von der in der katalytisch aktiven Multielementoxidmasse (III) enthaltenem Mo zu in der katalytisch aktiven Multielemetoxidmasse (III) enthaltenem V, Mo/V, 15:1 bis 1:1 beträgt, das entsprechende molare Verhältnis Mo/Cu 30:1 bis 1:3 und das entsprechende molare Verhältnis Mo/(Gesamtmenge aus W und Nb) 80:1 bis 1:4 beträgt.

Bevorzugte Multielementoxidaktivmassen III sind solche, deren Bereiche A eine Zusammensetzung im nachfolgenden Stöchiometrieraster der allgemeinen Formel IV aufweisen

Mo₁₂VₐX¹_{b}X²_{c}X⁵_{f}X⁶_{g}Oₓ (IV),

mit
- X¹: = W und/oder Nb,
- X²: = Cu und/oder Ni,
- X⁵: = Ca und/oder Sr,
- X⁶: = Si und/oder Al,
- a: = 2 bis 6,
- b: = 1 bis 2,
- c: = 1 bis 3,
- f: = 0 bis 0,75,
- g: = 0 bis 10, und
- x: = eine Zahl die durch die Wertigkeit und Häufigkeit der von Sauerstoff verschiedenen Elemente in (IV) bestimmt wird.

Der im Zusammenhang mit den Multielementoxidaktivmassen III verwendete Begriff "Phase" meint dreidimensional ausgedehnte Bereiche, deren chemische Zusammensetzung von der ihrer Umgebung verschieden ist. Die Phasen sind nicht notwendigerweise röntgenographisch homogen. In der Regel bildet die Phase A eine kontinuierliche Phase, in der Partikel der Phase B und gegebenenfalls C dispergiert sind.

Die feinteiligen Phasen B und gegebenenfalls C bestehen mit Vorteil aus Partikeln, deren Größendurchmesser, d.h., die längste durch den Schwerpunkt der Partikel gehende Verbindungsstrecke zweier auf der Oberfläche der Partikel befindlicher Punkte bis zu 300 µm, vorzugsweise 0,1 bis 200 µm, besonders bevorzugt 0,5 bis 50 µm und ganz besonders bevorzugt 1 bis 30 µm beträgt. Geeignet sind aber auch Partikel mit einem Größendurchmesser von 10 bis 80 µm oder 75 bis 125 µm.

Prinzipiell können die Phasen A, B und gegebenenfalls C in den erfindungsgemäß erhältlichen Multielementoxidativmassen III amorph und/oder kristallin vorliegen.

Günstig ist es, wenn die Phase B aus Kristalliten von Oxometallaten besteht oder solche Oxometallatkristallite (= Oxidkristallite) enthält, die das Röntgenbeugungsmuster und damit den Kristallstrukturtyp wenigstens eines der nachfolgenden Kupfermolybdate aufweisen. In Klammern ist die Fundstelle für den zugehörigen Röntgenbeugungsfingerabdruck angegeben.
- Cu₄Mo₆O₂₀: [A. Moini et al., Inorg. Chem. 25 (21) (1986) 3782-3785],
- Cu₄Mo₅O₁₇: [Karteikarte 39-181 der JCPDS-ICDD Kartei (1991)],
- a-CuMoO₄: [Karteikarte 22-242 der JCPDS-ICDD Kartei (1991)],
- Cu₆Mo₅O₁₈: [Karteikarte 40-865 der JCPDS-ICDD Kartei (1991)],
- Cu₄₋ₓMo₃O₁₂: mit X = 0 bis 0,25 [Karteikarte 24-56 und 26-547 der JCPDS-ICDD Kartei (1991)],
- Cu₆Mo₄O₁₅: [Karteikarte 35-17 der JCPDS-ICDD Kartei (1991)],
- Cu₃(MoO₄)₂(OH)₂: [Karteikarte 36-405 der JCPDS-ICDD Kartei (1991)],
- Cu₃Mo₂O₉: [Karteikarte 24-55 und 34-637 der JCPDS-ICDD Kartei (1991)],
- Cu₂MoO₅: [Karteikarte 22-607 der JCPDS-ICDD Kartei (1991)].

Vorzugsweise enthält die Phase B Oxometallate, die das Röntgenbeugungsmuster und damit den Kristallstrukturtyp des nachfolgenden Kupfermolybdats aufweisen:
- CuMoO₄ - III: mit Wolframit-Struktur gemäß Russian Journal of Inorganic Chemistry 36 (7) (1991) 927-928, Tabelle 1.

Unter diesen sind diejenigen mit der nachfolgenden Stöchimetrie V

Cu₁Mo_{A}W_{B}V_{C}Nb_{D}Ta_{E}O_{y}•(H₂O)_{F} (V),

mit
- 1/ (A+B+C+D+E):: 0,7 bis 1,3 vorzugsweise 0,85 bis 1,15, besonders bevorzugt 0,95 bis 1,05 und ganz besonders bevorzugt 1,
- F:: 0 bis 1,
- B+C+D+E:: 0 bis 1, vorzugsweise 0 bis 0,7, und
- Y: eine Zahl, die durch die Wertigkeit und Häufigkeit der von Sauer- stoff verschiedenen Elemente bestimmt wird,
bevorzugt. IV

Besonders bevorzugt sind unter diesen diejenigen der Stöchiometrien VI, VII oder VIII:

Cu₁Mo_{A}W_{B}V_{C}O_{y} (VI),

mit
- 1/ (A+B+C):: 0,7 bis 1,3, vorzugsweise 0,85 bis 1,15, besonders bevorzugt 0,95 bis 1,05 und ganz besonders bevorzugt 1,
- B+C:: 0 bis 1, vorzugsweise 0 bis 0,7, und
- y: eine Zahl, die durch die Wertigkeit und Häufigkeit der von Sauer- stoff verschiedenen Elemente bestimmt wird;

Cu₁Mo_{A}W_{B}O_{y} (VI),
mit
- 1/ (A+B):: 0,7 bis 1,3, vorzugsweise 0,85 bis 1,15, besonders bevorzugt 0,95 bis 1,05 und ganz besonders bevorzugt 1,
- A, B:: 0 bis 1 und
- y: eine Zahl, die durch die Wertigkeit und Häufigkeit der von Sauer- stoff verschiedenen Elemente bestimmt wird;

Cu₁Mo_{A}W_{B}O_{y} (VIII),
mit
- 1/ (A+C):: 0,7 bis 1,3, vorzugsweise 0,85 bis 1,15, besonders bevorzugt 0,95 bis 1,05 und ganz besonders bevorzugt 1,
- A, C:: 0 bis 1 und
- y: eine Zahl, die durch die Wertigkeit und Häufigkeit der von Sauer- stoff verschiedenen Elemente bestimmt wird.

Die Herstellung solcher Oxometallate offenbart beispielsweise die EP-A 668 104.

Geeignete Phasen B sind auch solche, die Oxometallate der nachfolgenden Stöchiometrie IX

Cu₁Mo_{A}W_{B}V_{C}Nb_{D}Ta_{E}O_{y} (IX),

mit
- 1 (A+B+C+D+E):: 0,7 bis 1,3, vorzugsweise 0,85 bis 1,15, besonders bevorzugt 0,95 bis 1,05 und ganz besonders bevorzugt 1,
- (B+C+D+E)/A:: 0,01 bis 1, vorzugsweise 0,05 bis 0,3, besonders bevorzugt 0,075 bis 0,15 und ganz besonders bevorzugt 0,11 und
- y: eine Zahl, die durch die Wertigkeit und Häufigkeit der von Sauer- stoff verschiedenen Elemente bestimmt wird,
und des als HT-Kupfermolybdat-Struktur bezeichneten Strukturtyps enthalten, der durch ein Röntgenbeugungsmuster (Fingerabdruck) gekennzeichnet ist, dessen charakteristischste und intensivste Beugungslinien, angegeben als Netzebenenabstände d [Å], wie folgt sind:
6,79 ± 0,3
3,56 ± 0,3
3,54 ± 0,3
3,40 ± 0,3
3,04 ± 0,3
2,96 ± 0,3
2,67 ± 0,2
2,66 ± 0,2
2,56 ± 0,2
2,36 ± 0,2
2,35 ± 0,2
2,27 ± 0,2
2,00 ± 0,2
1,87 ± 0,2
1,70 ± 0,2
1,64 ± 0,2
1,59 ± 0,2
1,57 ± 0,2
1,57 ± 0,2
1,55 ± 0,2
1,51 ± 0,2
1,44 ± 0,2.

Für den Fall, dass die Phase B ein Gemisch aus verschiedenen Oxometallaten enthält, ist ein Gemisch von Oxometallaten mit Wolframit- und HT-Kupfermolybdat-Struktur bevorzugt. Das Gewichtsverhältnis von Kristalliten mit HT-Kupfermolybdat-Struktur zu Kristalliten mit Wolframit-Struktur kann dabei 0,01 bis 100, 0,1 bis 10, 0,25 bis 4 sowie 0,5 bis 2 betragen.

Die Herstellung von Oxometallaten IX offenbart z.B. die DE-A 195 28 646.

Die Phase C besteht vorzugsweise aus Kristalliten, die den Trirutil-Strukturtyp des a-und/oder β-Kupferantimonats CuSb₂O₆ aufweisen. α-CuSb₂O₆ kristallisiert in einer tetragonalen Trirutil-Struktur (E.-O. Giere et al., J. Solid State Chem. 131 (1997) 263-274), während β-CuSb₂O₆ eine monoklin verzerrte Trirutil-Struktur aufweist (A. Nakua et al., J. Solid State Chem. 91 (1991) 105-112 oder Vergleichsdiffraktogramm in Karteikarte 17-284 in der JCPDS-ICDD-Kartei 1989). Darüber hinaus werden Phasen C bevorzugt, die die Pyrochlore-Struktur des Minerals Parzite, eines Kupfer-Antimon-Oxid-Hydroxyds mit der variablen Zusammensetzung CuySb₂₋ₓ(O,OH, H₂O)₆₋₇(y≤ 2,0 ≤x≤1) aufweisen (B. Mason et al., Mineral. Mag. 30 (1953) 100-112 oder Vergleichsdiagramm in Karteikarte 7-303 der JCPDS-ICDD-Kartei 1996).

Des weiteren kann die Phase C aus Kristalliten bestehen, die die Struktur des Kupferantimonats Cu₉Sb₄O₁₉ (S. Shimada et al., Chem. Lett. 1983, 1875-1876 oder S. Shimada et. al., Thermochim. Acta 133 (1988) 73-77 oder Vergleichsdiagramm in Karteikarte 45-54 der JCPDS-ICDD-Kartei) und/oder die Struktur von Cu₄SbO_{4,5} (S. Shimada et al., Thermochim. Acta 56 (1982)73-82 oder S. Shimada et al., Thermochim. Acta 133 (1988) 73-77 oder Vergleichsdiagramm in Karteikarte 36-1106 der JCPDS-ICDD-Kartei) aufweisen.

Selbstverständlich können die Bereiche C auch aus Kristalliten bestehen, die ein Gemenge aus den vorgenannten Strukturen darstellen.

Die den Multielementoxidaktivmassen der allgemeinen Formel III zugrunde liegenden und erfindungsgemäß thermisch zu behandelnden innigen Trockengemischen können z.B. so erhalten werden, wie es in den Schriften WO 02/24327, DE-A 4405514, DE-A 4440891, DE-A 19528646, DE-A 19740493, EP-A 756894, DE-A 19815280, DE-A 19815278, EP-A 774297, DE-A 19815281, EP-A 668104 und DE-A 19736105 beschrieben ist. Erfindungsgemäß ist lediglich die Miteinarbeitung von Ammoniumionen zu berücksichtigen.

D.h., alle in den vorgenannten Schriften beispielhaften Ausführungsformen erzeugten innigen Trockengemische können erfindungsgemäß thermisch behandelt werden und führen zu unmittelbaren Verfahrensprodukten, die in hervorragender Weise für Katalysatoren zur heterogen katalysierten partiellen Gasphasenoxidation von Acrolein zu Acrylsäure geeignet sind.

Das Grundprinzip der Herstellung von innigen Trockengemischen, die bei erfindungsgemäßer Behandlung zu vorteilhaften Multielementoxidativmassen der allgemeinen Formel III führen, besteht darin, wenigstens eine Multielementoxidmasse B (X₁⁷CuₕHᵢO_{y}) als Ausgangsmasse 1 und gegebenenfalls eine oder mehrere Multielementoxidmassen C (X₁⁸SbⱼHₖO_{z}) als Ausgangsmasse 2 entweder voneinander getrennt oder miteinander vergesellschaftet in feinteiliger Form vorzubilden und anschließend die Ausgangsmassen 1 und gegebenenfalls 2 mit einem Gemisch, das Quellen der elementaren Konstituenten der Multielementoxidmasse A

Mo₁₂VₐX_{b}¹X_{c}²X_{d}³Xₑ⁴X_{f}⁵X_{g}⁶Oₓ (A),

in einer der Stöchiometrie A entsprechenden Zusammensetzung enthält, im gewünschten Mengenverhältnis (gemäß der allgemeinen Formel III) in innigen Kontakt zu bringen und die dabei resultierende innige Mischung gegebenenfalls zu trocknen. Erfindungsgemäß wesentlich ist dabei nur, dass entweder die Quellen der elementaren Konstituenten der Multielementoxidmasse A Ammoniumionen enthalten, und/oder beim innigen in Kontakt bringen vollständig zersetzliche, Ammoniumionen enthaltende, Salze wie NH₄NO₃, NH₄Cl, Ammoniumacetet etc. zugegeben werden.

Das innige in Kontakt bringen der Bestandteile der Ausgangsmassen 1 sowie gegebenenfalls 2 mit dem die Quellen der elementaren Konstituenten der Multimetalloxidmasse A enthaltenden Gemisch (Ausgangsmasse 3) kann sowohl trocken als auch nass erfolgen. Im letzteren Fall muss lediglich darauf geachtet werden, dass die vorgebildeten Phasen (Kristallite) B und gegebenenfalls C nicht in Lösung gehen. In wässrigem Medium ist letzteres bei pH-Werten, die nicht zu stark von 7 abweichen und bei nicht zu hohen Temperaturen üblicherweise gewährleistet. Erfolgt das innige in Kontakt bringen nass, wird abschließend normalerweise zum erfindungsgemäß thermisch zu behandelnden innigen Trockengemisch getrocknet (z.B. durch Sprühtrocknen). Im Rahmen eines trockenen Mischens fällt eine solche Trockenmasse automatisch an. Selbstredend können die feinteilig vorgebildeten Phasen B und gegebenenfalls C auch in ein plastisch verformbares Gemisch, das die Quellen der elementaren Konstituenten der Multimetalloxidmasse A enthält, eingearbeitet werden, wie es die DE-A 10046928 empfiehlt. Natürlich kann das innige in Kontakt bringen der Bestandteile der Ausgangsmassen 1 sowie gegebenenfalls 2 mit den Quellen der Multielementoxidmasse A (Ausgangsmasse 3) auch so erfolgen, wie es die DE-A 19815281 beschreibt.

Erfindungsgemäß zweckmäßig kann man auch so vorgehen, dass man die Quellen der Multielementoxidmasse A innig mischt (z.B. in wässrigem Medium lösen und/oder suspendieren und anschließend das wässrige Gemisch sprühtrocknen) und die dabei resultierende feinteilige Ausgangsmasse 3 mit der feinteiligen Ausgangsmasse 1 sowie gegebenenfalls 2 vermengt und unter Zusatz von Wasser sowie gegebenenfalls sonstigem Plastifizierungsmittel miteinander verknetet. Das Vermengen kann in einer speziellen Mischvorrichtung außerhalb des Kneters oder auch im Kneter selbst (Änderung der Laufrichtung) erfolgen. Letzteres ist vorteilhaft. Die Knetmasse wird anschließend extrudiert und die extrudierten Stränge getrocknet. Nachfolgend können die extrudierten Stränge wie beschrieben erfindungsgemäß thermisch behandelt werden. Das dabei anfallende Calcinationsgut wird danach normalerweise gemahlen und wie in der EP-A 714700 beschrieben zur Herstellung von Schalenkatalysator verwendet.

Als Quellen der Multielementoxidmasse A kommen prinzipiell all jene in Betracht, die auch als Quellen für die Multielementoxidmassen I geeignet sind.

Als Plastifizierungsmittel sind dabei im wesentlichen alle Lösungsmittel geeignet, die bei Temperaturen bis etwa 360°C weitgehend rückstandsfrei verdampfen oder sich weitgehend rückstandfrei zersetzen.

Das Plastifizierungsmittel wird in zweckmäßiger Weise so gewählt, dass es das feinteilige Trockengemisch aus den Ausgangsmassen 1, 3 und gegebenenfalls 2 gut benetzt. Geeignete Plastifizierungsmittel sind neben Wasser Carbonsäuren, die verzweigt oder geradkettig, gesättigt oder ungesättigt sein können, wie z.B. Ameisensäure und Essigsäure, primäre oder sekundäre C₁- bis C₈-Alkohole, wie Methanol, Ethanol, 2-Propanol, aber auch Aldehyde oder Ketone wie Aceton und Gemische davon.

Als Kneter können z.B. kontinuierliche Schneckenkneter oder Trogkneter verwendet werden. Kontinuierliche Schneckenkneter weisen eine oder mehrerer achsparallele, in einem zylindrischen Gehäuse angeordnete Schnecken auf, die auf einer Welle Knet- und Transportelemente aufweisen, die das an einem Ende des Kneters aufgegebene Material zum Austrittsende des Kneters befördern und gleichzeitig plastifizieren und homogenisieren. Anwendungstechnisch zweckmäßig sind Trogkneter mit wenigstens zwei horizontal gelagerten Rotoren, z.B. ein sogenannter doppelschaufliger Trogkneter mit zwei gegenläufig rotierbaren Knetschaufeln in einem Doppelmuldentrog. Die Rotoren können unterschiedliche Formen wie Sigma-, Mastikator-, Nabenform usw. aufweisen. Die Kneter können chargenweise oder kontinuierlich arbeiten. Alternativ kann man auch schnelllaufende Intensivmischer wie Pflugscharmischer oder Schräglagemischer oder einen vergleichsweise langsam laufenden Simpsonmischer verwenden, die gegebenenfalls mit hochtourig laufenden Messerelementen ausgerüstet sind.

Es ist günstig, wenn das Verkneten bei Temperaturen von weniger als 90°C, vorzugsweise weniger als 80°C und besonders bevorzugt weniger als 60°C erfolgt. In der Regel wird die Temperatur beim Verkneten mehr als 0°C und meist 20 bis 45°C betragen.

Weiterhin ist es günstig, wenn das Verkneten weniger als 10 Stunden, vorzugsweise weniger als 3 Stunden und besonders bevorzugt weniger als 1 Stunde in Anspruch nimmt. In der Regel wird das Verkneten mehr als 15 min in Anspruch nehmen.

Die nach dem Verkneten erhaltene plastische ("plastisch" bzw. "pastenförmig" bezeichnet eine Konsistenz die kohärent ist und nicht wie ein Pulver aus diskreten Teilchen besteht und erst unter Einwirkung einer bestimmten Kraft verformbar ist und nicht, wie eine Lösung oder Suspension, bereitwillig die Gestalt eines Gefäßes annimmt) Masse kann unmittelbar zu Formkörpern beliebiger Geometrie geformt und diese Formkörper nach ihrer Trocknung erfindungsgemäß thermisch behandelt werden, wodurch unmittelbar Vollkatalysatoren erhalten werden können. Hierzu ist die Anwendung eines Extruders (alternativ kann ein Alexanderwerk oder eine Strangpresse verwendet werden) besonders geeignet. Häufig wird das Extrudat eine Strangform mit einem Durchmesser von z.B. 1 bis 20 mm, häufig 3 bis 10 mm, auf einer Länge von z.B. 0,5 bis 20 cm aufweisen. Die Stränglinge können dann, wie bereits beschrieben, getrocknet, thermisch behandelt, gemahlen und das Mahlgut zu Schalenkatalysatoren verarbeitet werden. Die Trocknung wird in der Regel bei 50 bis 180°C, meist bei etwa 120 bis 130°C (in zweckmäßiger Weise im Luftstrom) durchgeführt werden.

Generell bestehen die feinteiligen Ausgangsmassen 1, 2 erfindungsgemäß vorteilhaft aus Partikeln, deren Größtdurchmesser d (längste durch den Schwerpunkt der Partikel gehende Verbindungsstrecke zweier auf der Oberfläche der Partikel befindlicher Punkte)>0 bis 300 µm, vorzugsweise 0,01 bis 100 µm und besonders bevorzugt 0,05 bis 20 µm beträgt. Selbstverständlich können die Partikeldurchmesser d aber auch 0,01 bis 150 µm oder 0,5 bis 50 µm betragen.

Es ist möglich, dass die erfindungsgemäß zu verwendenden Ausgangsmassen 1, 2 eine spezifische Oberfläche O (bestimmt nach DIN 66131 durch Gasadsorption (N₂) gemäß Brunner-Emmert-Teller (BET)) aufweisen, die ≤80 m²/g, häufig ≤50 m²/g oder ≤ 10m²/g und teilweise ≤ m²/g beträgt. In der Regel wird 0>0,1 m²/g betragen.

Prinzipiell können die Ausgangsmassen 1, 2 erfindungsgemäß sowohl amorph und/oder kristallin vorliegend eingesetzt werden.

Günstig ist es, wenn die Ausgangsmassen 1,2 aus Kristalliten von Oxometallaten B (z.B. solchen der allgemeinen Formeln V bis IX) und Kristalliten von Oxometallaten C, wie sie vorstehend beschrieben wurden, bestehen. Wie bereits angeführt, sind solche Oxometallate B z.B. nach den Verfahrensweisen der EP-A 668 104 bzw. der DE-A 19 528 646 erhältlich. Aber auch die Herstellverfahren der DE-A 44 05 514 und der DE-A 44 40 891 sind anwendbar.

Prinzipiell können Oxometallate B enthaltende bzw. aus Oxometallaten B bestehende Multielementoxidmassen B in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes Trockengesmich erzeugt und dieses bei Temperaturen (Materialguttemperaturen) von 200 bis 1000°C, vorzugsweise 250 bis 900°C bzw. 700 bis 850°C mehrere Stunden unter Intertgas, z.B. Stickstoff, einem Gemisch aus Inertgas und Sauerstoff oder bevorzugt an der Luft calciniert, wobei die Calcinationsdauer einige Minuten bis einige Stunden betragen kann. Dabei kann die Calcinationsatmosphäre zusätzlich Wasserdampf enthalten. Eine Calcination unter reinem Sauerstoff ist ebenfalls möglich. Als Quellen für die elementaren Konstituenten der Multimetalloxidmasse B kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind. Neben den Oxiden kommen als solche Ausgangsverbindungen vor allem Halogenide, Nitrate, Formiate, Oxalate, Citrate, Acetate, Carbonate, Amminkomplexsalze, Ammoniumsalze und/oder Hydroxide in Betracht (Verbindungen wie NH₄OH, (NH₄)₂CO₃, NH₄NO₃, NH₄CHO₂, CH₃COOH, NH₄CH₃CO₂ oder Ammoniumoxalat, die spätestens beim späteren Calcinieren zu gasförmig entweichenden Verbindungen zerfallen und/oder zersetzt werden können, können zusätzlich eingearbeitet werden).

Das innige Vermischen der Ausgangsverbindungen zur Herstellung von Multielementoxidmassen B kann in trockener Form oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolg das innige Vermischen jedoch in nasser Form. Üblicherweise werden dabei die Ausgangsverbindungen in Form einer wässrigen Lösung und/oder Suspension miteinander vermischt. Besonders innige Trockengemische werden beim beschriebenen Trockenverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt. Anschließend wird die getrocknete Masse wie oben bereits beschrieben calciniert.

Eine bevorzugte Herstellweise von Oxometallaten B (X⁷₁CuₕHⱼO_{y} mit X = Mo u./o. W) besteht darin, eine wässrige Lösung von Ammoniumheptamolybdat und Ammoniumparawolframat mit einer wässrigen ammoniakalischen Lösung von Kupfercarbonat (z.B. der Zusammensetzung Cu(OH)₂CO₃ bzw. Kupferacetat und/oder Kupferformiat zu versetzen, die resultierende Mischung zu trocknen, z.B. sprühzutrocknen, und das erhaltene Trockengemisch, gegebenenfalls nach einer anschließenden Verknetung und Verstrangung sowie Trocknung, wie beschrieben zu calcinieren.

In einer anderen Herstellvariante der Multielementoxidmassen B erfolgt die thermische Behandlung des Gemisches der verwendeten Ausgangsverbindungen in einem Über druckgefäß (Autoklav) in Gegenwart von überatmosphärischen Druck aufweisendem Wasserdampf bei Temperaturen im Bereich von > 100 bis 600°C. Der Druckbereich erstreckt sich in typischer Weise auf bis zu 500 atm, vorzugsweise auf bis zu 250 atm. Mit besonderem Vorteil erfolgt diese hydrothermale Behandlung im Temperaturbereich von > 100 bis 374,15°C (kritische Temperatur des Wassers), in dem Wasserdampf und flüssiges Wasser unter den sich einstellenden Drucken koexistieren.

Die wie ebenda beschriebenen erhältlichen Multielementoxidmassen B, die Oxometallate B eines einzelnen Strukturtyps oder eine Mischung von Oxometallaten B verschiedener Strukturtypen enthalten können, oder ausschließlich aus Oxometallaten B eines einzelnen Strukturtyps oder aus einer Mischung von Oxometallaten verschiedener Strukturtypen bestehen, können nun, gegebenenfalls nach Mahlung und/oder Klassierung auf gewünschte Größen, als erfindungsgemäß benötigte Ausgangsmasse 1 eingesetzt werden.

Die Multimetalloxidmassen C können prinzipiell in derselben Weise hergestellt werden wie die Multimetalloxidmassen III. Die Calcination des innigen Trockengemisches erfolgt im Fall der Multimetalloxidmasse C zweckmäßigerweise bei Temperaturen (Materialguttemperaturen) von 250 bis 1200°C, vorzugsweise von 250 bis 850°C. Die Calcination kann unter Inertgas, z.B. Stickstoff, aber auch unter einem Gemisch aus Inertgas und Sauerstoff wie z.B. Luft oder auch unter reinem Sauerstoff erfolgen. Eine Calcination unter einer reduzierenden Atmosphäre ist ebenfalls möglich. Als solche reduzierend wirkenden Gase können z.B. Kohlenwasserstoffe wie Methan, Aldehyde wie z.B. Acrolein oder Ammoniak verwendet werden. Die Calcination kann aber auch unter einem Gemisch aus O₂ und reduzierend wirkenden Gasen erfolgen, wie es beispielsweise in der DE-A 4335973 beschrieben ist. Bei einer Calzination unter reduzierenden Bedingungen ist allerdings zu beachten, dass die metallischen Konstituenten nicht bis zum Element reduziert werden. In der Regel nimmt die erforderliche Calcinationsdauer (in der Regel einige Stunden) auch hier mit zunehmender Calcinierungstemperatur ab.

Erfindungsgemäß bevorzugt werden Multielementoxidmassen C verwendet, die dadurch erhältlich sind, dass man von Quellen der elementaren Konstituenten der Multielementoxidmasse C, die wenigstens einen Teil, vorzugsweise die Gesamtmenge, des Antimons in der Oxidationsstufe +5 enthalten, ein Trockengemisch herstellt und dieses bei Temperaturen (Materialguttemperaturen) von 200 bis 1200°C, vorzugsweise von 200 bis 850°C und besonders bevorzugt von 300 bis 600°C calciniert. Derartige Multimetalloxidmassen C sind z. B. nach den in der DE-A 24 07 677 ausführlich beschriebenen Herstellweisen erhältlich. Unter diesen ist diejenige Verfahrensweise bevorzugt, bei der man Antimontrioxid oder Sb₂O₄ in wässrigem Medium mittels Wasserstoffperoxid in einer Menge, die unterhalb der stöchiometrischen liegt oder ihr gleich ist oder diese übersteigt, bei Temperaturen zwischen 40 und 100°C zu Antimon(V)oxidhydroxid oxidiert, bereits vor dieser Oxidation, während dieser Oxidation und/oder nach dieser Oxidation wässrige Lösungen und/oder Suspensionen von geeigneten Ausgangsverbindungen der übrigen elementaren Konstituenten der Multimetalloxidmasse C zusetzt, anschließend das resultierende wässrige Gemisch trocknet (vorzugsweise sprühtrocknet, Eingangstemperatur: 200 bis 300°C, Ausgangstemperatur: 80 bis 130°C, häufig 105 bis 115°C) und danach das innige Trockengemisch wie beschrieben calciniert.

Bei dem wie eben beschriebenen Verfahren kann man z.B. wässrige Wasserstoffperoxidlösungen mit einem H₂O₂-Gehalt von 5 bis 33 Gew.-% verwenden. Eine nachträgliche Zugabe von geeigneten Ausgangsverbindungen der übrigen elementaren Konstituenten des Oxometallats C ist vor allem dann zu empfehlen, wenn diese das Wasserstoffperoxid katalytisch zu zersetzen vermögen. Selbstverständlich könnte man aber auch das Antimon(V)oxidhydroxid aus dem wäsrigen Medium isolieren und z.B. mit geeigneten feinteiligen Ausgangsverbindungen der übrigen elementaren Konstituenten des Oxometallats C sowie gegebenenfalls weiteren Sb-Ausgangsverbindungen innig vermischen und anschließend dieses innige Gemisch wie beschrieben calcinieren.

Wesentlich ist, dass es sich bei den Elementquellen der Oxometallate C, entweder bereits um Oxide handelt, oder um solche Verbindungen, die durch Erhitzen, gegebenenfalls in Anwesenheit von Sauerstoff, in Oxide überführbar sind. Neben den Oxiden kommen daher als Ausgangsverbindungen vor allem Halogenide, Nitrate, Formiate, Oxalate, Acetate, Carbonate und/oder Hydroxide in Betracht (Verbindungen wie NH₄OH, (NH₄)₂CO₃ NH₄NO₃, NH₄CHO₂, CH₃COOH, NH₄CH₃CO₂ oder Ammoniumoxalat, die spätestens beim späteren Calcinieren zu gasförmig entweichenden Verbindungen zerfallen und/oder zersetzt werden können, können zusätzlich eingearbeitet werden).

Generell kann auch zur Herstellung von Oxometallaten C das innige Vermischen der Ausgangsverbindungen in trockener oder in nasser Form erfolgen. Erfolgt es in trockener Form, werden die Ausgangsverbindungen zweckmäßigerweise als feinteilige Pulver eingesetzt. Vorzugsweise erfolgt das innige Vermischen jedoch in nasser Form. Üblicherweise werden die Ausgangsverbindungen dabei in Form einer wässrigen Lösung und/oder Suspension miteinander vermischt. Nach Abschluss des Mischvorgangs wird die fluide Masse getrocknet und nach Trocknung calciniert. Vorzugsweise erfolgt auch hier die Trocknung durch Sprühtrocknung. Nach erfolgter Calcinierung können die Oxometallate C nochmals zerkleinert (z.B. durch Nass- oder Trockenmahlen, z.B. in der Kugelmühle oder durch Strahlmahlen) und aus dem dabei erhältlichen, in der Regel aus im wesentlichen kugelförmigen Partikeln bestehenden, Pulver die Kornklasse mit einem im für das erfindungsgemäß erhältliche Multielementoxid (III) gewünschten Größtdurchmesserbereich liegenden Korngrößtdurchmesser durch in an sich bekannter Weise durchzuführendes Klassieren (z.B. Nass- oder Trockensiebung) abgetrennt werden. Eine bevorzugte Herstellweise von Oxometallaten C der allgemeinen Formel (Cu, Zn)₁SbₕHᵢO_{y} besteht darin, Antimontrioxid und/oder Sb₂O₄ in wässrigem Medium mittels Wasserstoffperoxid zunächst in eine, vorzugsweise feinteilige, Sb (V)-Verbindung, z.B. Sb (V)-Oxidhydroxidhydrat, überzuführen, die dabei resultierende wässrige Suspension mit einer ammoniakalischen wässrigen Lösung von Zn- und/oder Cu-Carbonat (das z.B. die Zusammensetzung Cu₂(OH)₂CO₃ aufweisen kann) bzw. Zn- und/oder Cu-Acetat und/oder -Formiat zu versetzen, die resultierende wässrige Mischung zu trocknen, z. B. wie beschrieben sprühzutrocknen, und das erhaltene Pulver, gegebenenfalls nach anschließendem Verkneten mit Wasser und darauffolgendem Verstrangen und Trocknen, wie beschrieben zu calcinieren. Unter günstigen Umständen können die Oxometallate B und die Oxometallate C in miteinander vergesellschafteter Form hergestellt werden. In diesen Fällen wird ein Gemisch aus Kristallisaten der Oxometallate B und Kristalliten der Oxometallate C erhalten, das unmittelbar als Ausgangsmasse 1 + 2 eingesetzt werden kann.

Zur Herstellung einer als Ausgangsmasse 3 geforderten wässrigen Lösung bedarf es ausgehend von den bereits genannten Quellen der elementaren Konstituenten in der Regel der Anwendung erhöhter Temperaturen. In der Regel werden Temperaturen ≥60°C, meist ≥70°C, im Normalfall jedoch ≤100°C angewendet. Letzteres und das Nachfolgende gilt insbesondere dann, wenn als Mo-Elementquelle das Ammoniumheptamolybdattetrahydrat [AHM = (NH₄)₆Mo₇O₂₄•4H₂O] und/oder als Vanadinquelle Ammoniummetavanadat [AMV = NH₄VO₃] verwendet wird. Als besonderes schwierig gestalten sich die Verhältnisse dann, wenn das Element W Bestandteil der wässrigen Ausgangsmasse 3 ist und Ammoniumparawolframatheptahydrat [APW = (NH₄)₁₀W₁₂O₄₁•7H₂O] neben wenigstens einer der beiden vorgenannten Elementquellen als Ausgangsverbindung der relevanten wässrigen Lösung eingesetzt wird.

Es wurde nun überraschend gefunden, dass bei erhöhten Temperaturen als Ausgangsmasse 3 hergestellte wässrige Lösungen beim und nach dem anschließenden Abkühlen unter die Lösetemperatur, selbst bei Gehalten des Elementes Mo von ≥10 Gew.-% und Abkühltemperaturen von bis zu 20°C oder darunter (meist nicht < 0°C), bezogen auf die wässrige Lösung, im Normalfall stabil sind. D.h., beim oder nach dem Abkühlen der wässrigen Lösung fällt kein Feststoff aus. Vorgenannte Aussage gilt in der Regel auch noch bei entsprechend bezogenen Mo-Gehalten von bis zu 20 Gew.-%. Ähnliches gilt für V und W.

Üblicherweise beträgt der Mo-Gehalt von solchen auf Temperaturen von bis zu 20°C oder darunter (meist nicht unter 0°C) abgekühlten, als Ausgangsmasse 3 geeigneten, wässrigen Lösungen, auf die Lösungen bezogen, nicht mehr als 35 Gew.-%.

Vorstehender Befund wird darauf zurückgeführt, dass beim Lösen bei erhöhter Temperatur offensichtlich Verbindungen der relevanten Elemente entstehen, die eine erhöhte Wasserlöslichkeit aufweisen. Diese Vorstellung wird dadurch gestützt, dass auch der aus einer solchen wässrigen Lösung durch Trocknung erhältliche Rückstand (z.B. Sprühtrocknung) eine in entsprechender Weise erhöhte (auch bei den entsprechenden tiefen Temperaturen) Löslichkeit in Wasser aufweist.

Zweckmäßigerweise wird daher wie folgt vorgegangen. Bei einer Temperatur T_{L} ≥60°C (z.B. bei bis zu 65°C, oder bei bis zu 75°C, oder bei bis zu 85°C, oder bei bis zu 95°C oder bei ≤100°C) wird eine als Ausgangsmasse 3 geeignete wässrige Lösung erzeugt. In diese wässrige Lösung werden dann nach Abkühlung auf eine Temperatur T_{E} < T_{L} die feinteiligen festen Ausgangsmassen 1, 2 eingearbeitet. Häufig wird T_{L} > 70°C und T_{E} ≤70°C, betragen. Bei Inkaufnahme von etwas geringeren Lösegeschwindigkeiten und geringeren Feststoffgehalten ist aber auch T_{L} ≤60°C möglich.

Die Einarbeitung der vorpräparierten festen Ausgangsmassen 1, 2 in die wässrige Ausgangsmasse 3 (wässrige Lösung oder wässrige Suspension oder mit Wasser angeteigte Masse) erfolgt üblicherweise durch Zugabe der Ausgangsmassen 1, 2 in die, wie bereits ausgeführt, abgekühlte wässrige Ausgangsmasse 3 und anschließendes mechanisches Vermischen, z.B. unter Verwendung von Rühr oder Dispergierhilfsmitteln, über einen Zeitraum von wenigen Minuten bis Stunden, bevorzugt in einem Zeitraum von 20 bis 40 min. Wie bereits ausgeführt, ist es erfindungsgemäß besonders günstig, wenn die Einarbeitung der festen Ausgangsmassen 1, 2 in die wässrige Ausgangsmasse 3 bei Temperaturen ≤70°C, bevorzugt bei Temperaturen ≤60°C, und besonders bevorzugt bei Temperaturen ≤40°C erfolgt. In der Regel wird die Einarbeitungstemperatur ≥0°C betragen.

Weiterhin ist es günstig, wenn die Einarbeitung der festen Ausgangsmassen 1, 2 in eine wässrige Ausgangsmasse 3 hinein erfolgt, deren pH-Wert bei 25°C 4 bis 7, bevorzugt 5 bis 6,5 beträgt. Letzteres kann z. B. dadurch erreicht werden, dass man der wässrigen Ausgangsmasse 3 ein oder mehrere pH-Puffersysteme zusetzt. Als solche eignet sich beispielsweise ein Zusatz von Ammoniak und Essigsäure und/oder Ameisensäure oder ein Zusatz von Ammoniumacetat und/ oder Ammoniumformiat. Selbstverständlich kann bezüglich des vorgenannten Verwendungszwecks auch Ammoniumcarbonat mit verwendet werden. Die Trocknung der bei Einarbeitung der Ausgangsmassen 1, 2 in die wässrige Ausgangsmasse 3 erhaltenen wässrigen Mischung erfolgt üblicherweise durch Sprühtrocknung. Dabei werden zweckmäßigerweise Austrittstemperaturen von 100 bis 150°C eingestellt. Wie immer in dieser Schrift kann sowohl im Gleichstrom als auch im Gegenstrom sprühgetrocknet werden.

Eigene Untersuchungen haben ergeben, dass beim erfindungsgemäßen thermischen Behandeln des die Ausgangsmassen 1, 3 und gegebenenfalls 2 enthaltenden innigen Trockengemischs der Strukturtyp der in den Ausgangsmassen 1, 2 enthaltenen Kristallite im wesentlichen erhalten bleibt, oder sich allenfalls in andere Strukturtypen umwandelt. Ein Verschmelzen (Ineinanderlösen) der Bestandteile der Ausgangsmassen 1, 2 untereinander oder mit jenen der Ausgangsmasse 3 findet jedoch im wesentlichen nicht statt.

Dies eröffnet, wie bereits oben angedeutet, die Möglichkeit, nach Mahlen der vorgebildeten Ausgangsmassen 1, 2 die Kornklasse mit einem im für die Multielementoxidmasse (III) gewünschten Größtdurchmesser (in der Regel > 0 bis 300 µm, vorzugsweise 0,01 bis 100 µm und besonders bevorzugt 0,05 bis 20 µm) durch in an sich bekannter Weise durchzuführendes Klassieren (z.B. Nass- oder Trockensiebung) abzutrennen und so zur Herstellung der gewünschten Multielementoxidmasse maßgeschneidert einzusetzen.

Prinzipiell können die erfindungsgemäß erhältlichen Multielementoxidmassen III ebenso wie die erfindungsgemäß erhältlichen Multielementoxidaktivmassen I, II in Pulverform als Katalysatoren für dieheterogen katalysierte partielle Gasphasenoxidation von Acrolein zu Acrylsäure eingesetzt werden (z.B. in einem Wirbelbett oder Fließbettreaktor).

Ebenso wie die Multielementoxidaktivmassen I, II werden aber auch die Multielementoxidaktivmassen III vorzugsweise zu Formkörpern bestimmter Geometrie geformt als Katalysatoren für die vorgenannte Partialoxidation eingesetzt. Die Formgebung und Auswahl der Geometrie kann dabei wie für die Multielementoxidaktivmassen I, II bereits beschrieben erfolgen.

D.h., entweder das innige, noch erfindungsgemäß thermisch zu behandelnde, Trockengemisch oder die aus selbigem erfindungsgemäß erhaltene Multielementoxidaktivmasse III selbst können auf vorgeformte inerte Katalysatorträger aufgetragen werden. Im ersteren Fall wird die erfindungsgemäße thermische Behandlung nach erfolgter Katalysatorträgerbeschichtung vorgenommen. Vorzugsweise wird nach der erfindungsgemäßen thermischen Behandlung auf den Katalysatorträger aufgebracht.

Dabei können die üblichen Trägermaterialien wie poröse oder unporöse Aluminiumoxide, Siliciumdioxid, Thoriumdioxid, Zirkondioxid, Siliciumcarbid oder Silicate wie magnesium- oder Aluminiumsilicat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist z.B. die Verwendung von im wesentlichen unporösen, oberflächenrauhen, kugelförmigen Trägem aus Steatit, deren Durchmesser 1 bis 8 mm, bevorzugt 4 bis 5 mm beträgt. Die Schichtdicke der Aktivmasse wird zweckmäßigerweise als im Bereich 50 bis 500 µm, bevorzugt im Bereich 150 bis 250 µm liegend, gewählt. Es können als Trägerkörper aber auch wie bereits beschrieben Hohlzylinder (Ringe) verwendet werden. Es sei an dieser Stelle darauf hingewiesen, dass bei der Herstellung solcher Schalenkatalysatoren zur Beschichtung der Trägerkörper die aufzubringende Pulvermasse bzw. der Trägerkörper in der Regel mit Bindemittel befeuchtet und nach dem Aufbringen, z.B. mittels heißer Luft, wieder getrocknet wird.

Die Beschichtung der Trägerkörper wird zur Herstellung der Schalenkatalysatoren in der Regel in einem geeigneten Behälter ausgeführt, wie er z.B. aus der DE-A 29 096 71 oder aus der EP-A 293 859 vorbekannt ist. Vorzugsweise wird wie in der EP-A 714700 beschrieben beschichtet und Form gegeben.

In geeigneter Weise kann das erfindungsgemäße Verfahren so angewendet werden, dass die resultierenden Multielementoxidaktivmassen (III) eine spezifische Oberfläche von 0,50 bis 150 m²/g, ein spezifisches Porenvolumen von 0,10 bis 0,90 cm³/g und eine solche Porendurchmesser-Verteilung aufweisen, dass auf die Durchmesserbereiche 0,1 bis < 1 µm, 1,0 bis < 10 µm und 10 µm bis 100 µm jeweils wenigstens 10 % des Porengesamtvolumens entfallen. Auch können die in der EP-A 293 859 als bevorzugt genannten Porendurchmesser-Verteilungen eingestellt werden.

Selbstverständlich können die erfindungsgemäßen Multielementoxidmassen (III) auch als Vollkatalysatoren betrieben werden. Diesbezüglich wird das die Ausgangsmassen 1, 2 und 3 umfassende innige Trockengemisch vorzugsweise unmittelbar zur gewünschten Katalysatorgeometrie verdichtet (z.B. mittels Tablettieren, Extrudieren oder Strangpressen), wobei gegebenenfalls an sich übliche Hilfsmittel, z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können, und erfindungsgemäß thermisch behandelt. Generell kann auch hier vor der Formgebung erfindungsgemäß thermisch behandelt werden. Bevorzugte Vollkatalysatorgeometrie sind Hohlzylinder mit einem Außendurchmesser und einer Länge von 2 bis 10 mm bzw. 3 bis 8 mm und einer Wandstärke von 1 bis 3 mm.

Die erfindungsgemäß erhältlichen Multielementoxidaktivmassen eignen sich insbesondere für Katalysatoren mit erhöhter Aktivität und Selektivität (bei vorgegebenem Umsatz) zur gasphasenkatalytischen Oxidation von Acrolein zu Acrylsäure. Normalerweise wird bei dem Verfahren Acrolein eingesetzt, das durch die katalytische Gasphasenpartialoxidation von Propen erzeugt wurde. In der Regel werden die Acrolein enthaltenden Reaktionsgase dieser Propenoxidation ohne Zwischenreinigung eingesetzt. Üblicherweise wird die gasphasenkatalytische Partialoxidation des Acroleins in Rohrbündelreaktoren als heterogene Festbettoxidation ausgeführt. Als Oxidationsmittel wird in an sich bekannter Weise Sauerstoff, zweckmäßigerweise mit inerten Gasen verdünnt (z.B. in Form von Luft), eingesetzt. Geeignete Verdünnungsgase sind z.B. N₂, CO₂, Kohlenwasserstoff, rückgeführte Reaktionsabgase und/oder Wasserdampf. In der Regel wird bei der Acroleinpartialoxidation ein Acrolein : Sauerstoff : Wasserdampf : Inertgas-Volumenverhältnis von 1 : (1 bis 3) : (0 bis 20) : (3 bis 30), vorzugsweise von 1 : (1 bis 3) : (0,5 bis 10) : (7 bis 18) eingestellt. Der Reaktionsdruck beträgt im allgemeinen 1 bis 3 bar und die Gesamtraumbelastung beträgt vorzugsweise 1000 bis 3500 NI/I/h. Typische Vielrohr-Festbettreaktoren sind z.B. in den Schriften DE-A 2830765, DE-A 2201528 oder US-A 3147084 beschrieben. Die Reaktionstemperatur wird üblicherweise so gewählt, dass der Acroleinumsatz bei einfachem Durchgang oberhalb von 90 %, vorzugsweise oberhalb von 98 %, liegt. Im Normalfall sind diesbezüglich Reaktionstemperaturen von 230 bis 330°C erforderlich.

Insbesondere eignen sich die erfindungsgemäß erhältlichen Multielementoxidaktivmassen und die sie enthaltenden Katalysatoren für die in der WO 00/53557 und in der DE-A 19910508 beschriebene Hochlastfahrweise. Sie eignen sich aber auch für die Verfahren der DE-A 10313214, DE-A 10313213, DE-A 10313211, DE-A 10313208und 10313209.

Neben der gasphasenkatalytischen Partialoxidation von Acrolein zu Acrylsäure vermögen die erfindungsgemäßen Verfahrensprodukte aber auch die gasphasenkatalytische Partialoxidation anderer organischer Verbindungen wie insbesondere anderer, vorzugsweise 3 bis 6 C-Atome aufweisender, Alkane, Alkanole, Alkanale, Alkene und Alkenole (z.B. Propylen, Methacrolein, tert.-Butanol, Methylether des tert.-Butanol, iso-Buten, iso-Butan oder iso-Butyraldehyd) zu olefinisch ungesättigten Aldehyden und/oder Carbonsäuren, sowie den entsprechenden Nitrilen (Ammoxidation, vor allem von Propen zu Acrylnitril und von iso-Buten bzw. tert.-Butanol zu Methacrylnitril) zu katalysieren. Beispielhaft genannt sei die Herstellung von Acrolein, Methacrolein und Methacrylsäure. Sie eignen sich aber auch zur oxidativen Dehydrierung paraffinischer bzw. olefinischer Verbindungen.

Zur Durchführung der erfindungsgemäßen thermischen Behandlung sowie zur Durchführung der Calcinationen im Rahmen der Herstellung der Ausgangsmassen 1 und 2 sind prinzipiell unterschiedlichste Ofentypen wie z.B. Hordenöfen, Drehrohröfen, Bandcalzinierer, Wirbelbettöfen oder Schachtöfen geeignet. Vorzugsweise werden dazu erfindungsgemäß Drehrohröfen eingesetzt.

### Beispiel 1

### 1. Allgemeine Beschreibung des zur erfindungsgemäßen thermischen Behandlung sowie zur Calcination im Rahmen der Herstellung von Ausgangsmassen 1 und 2 verwendeten Drehrohrofens

Eine schematische Darstellung des Drehrohrofens zeigt die dieser Schrift beiliegende Figur 1. Die nachfolgenden Bezugsziffern beziehen sich auf diese Figur 1.

Zentrales Element des Drehrohrofens ist das Drehrohr (1). Es ist 4000 mm lang und weist einen Innendurchmesser von 700 mm auf. Es ist aus Edelstahl 1.4893 gefertigt und weist eine Wanddicke von 10 mm auf.

Auf der Innenwand des Drehrohrofens sind Hublanzen angebracht, die eine Höhe von 5 cm und eine Länge von 23,5 cm aufweisen. Sie dienen primär dem Zweck, das thermisch im Drehrohrofen zu behandelnde Gut anzuheben und dadurch zu durchmischen.

Auf ein und derselben Höhe des Drehrohrofens sind um den Umfang äquidistant (jeweils 90° Abstand) jeweils vier Hublanzen angebracht (ein Quadrupel). Längs des Drehrohrofens befinden sich acht solcher Quadrupel (jeweils im Abstand von 23,5 cm). Die Hublanzen zweier benachbarter Quadrupel sitzen am Umfang gegeneinander versetzt. Am Anfang und am Ende Drehrohrofens (erste und letzte 23,5 cm) befinden sich keine Hublanzen.

Das Drehrohr rotiert frei in einem Quader (2), der vier in der Länge des Drehrohres aufeinanderfolgende, gleich lange, elektrisch beheizte (Widerstandsheizung) Heizzonen aufweist, von denen jede den Umfang des Drehrohrofens umschließt. Jede der Heizzonen kann den entsprechenden Drehrohrabschnitt auf Temperaturen zwischen Raumtemperatur und 850°C erhitzen. Die maximale Heizleistung jeder Heizzone beträgt 30 kW. Der Abstand zwischen elektrischer Heizzone und Drehrohraußenfläche beträgt etwa 10 cm. Am Anfang und am Ende ragt das Drehrohr ca. 30 cm aus dem Quader heraus.

Die Umdrehungsgeschwindigkeit kann zwischen 0 und 3 Umdrehungen pro Minute variabel eingestellt werden. Das Drehrohr ist sowohl links- als auch rechts-drehbar. Bei Rechtsdrehung verweilt das Materialgut im Drehrohr, bei Linksdrehung wird das Materialgut vom Eintrag (3) zum Austrag (4) gefördert. Der Neigungswinkel des Drehrohres zur Horizontalen kann zwischen 0° und 2° variabel eingestellt werden. Im diskontinuierlichen Betrieb liegt er faktisch bei 0°. Im kontinuierlichen Betrieb befindet sich die tiefstliegendste Stelle des Drehrohrs beim Materialgutaustrag. Eine Schnellkühlung des Drehrohres kann dadurch erfolgen, dass die elektrischen Heizzonen abgeschaltet und ein Ventilator (5) eingeschaltet wird. Dieser saugt durch im unteren Boden des Quaders befindliche Löcher (6) Umgebungsluft an, und fördert sie durch drei im Deckel befindliche Klappen (7) mit variabel einstellbarer Öffnung.

Der Materialguteintrag wird über eine Zellenradschleuse kontrolliert (Massenkontrolle). Der Materialgutaustrag wird, wie bereits erwähnt, über die Drehrichtung des Drehrohrs gesteuert.

Bei diskontinuierlichem Betrieb des Drehrohres kann eine Materialgutmenge von 250 bis 500 kg thermisch behandelt werden. Sie befindet sich dabei normalerweise ausschließlich im beheizten Teil des Drehrohres.

Von einer auf der zentralen Achse des Drehrohres liegenden Lanze (8) führen in Abständen von 800 mm insgesamt drei Thermoelemente (9) vertikal ins Materialgut. Sie ermöglichen die Bestimmung der Temperatur des Materialguts. In dieser Schrift wird unter Temperatur des Materialgutes das arithmetische Mittel der drei Thermoelementtemperaturen verstanden. Innerhalb des im Drehrohr befindlichen Materialguts beträgt die maximale Abweichung zweier gemessener Temperaturen erfindungsgemäß zweckmäßig weniger als 30°C, bevorzugt weniger als 20°C, besonders bevorzugt weniger als 10°C und ganz besonders bevorzugt weniger als 5 bzw. 3°C.

Durch das Drehrohr können Gasströme geführt werden, mittels derer sich die Calcinationsatmosphäre bzw. allgemein die Atmosphäre der thermischen Behandlung des Materialgutes einstellen lässt.

Der Erhitzer (10) bietet die Möglichkeit, den ins Drehrohr geführten Gasstrom vorab seines Eintritts ins Drehrohr auf die gewünschte Temperatur zu erwärmen (z.B. auf die für das Materialgut im Drehrohr gewünschte Temperatur). Die maximale Leistung des Erhitzers liegt bei 1 x 50 kW + 1 x 30 kW. Vom Prinzip her kann es sich beim Erhitzer (10) z.B. um einen indirekten Wärmetauscher handeln. Solch ein Erhitzer kann prinzipiell auch als Kühler genutzt werden. In der Regel handelt es sich aber um einen Elektroerhitzer, bei dem der Gasstrom über Strom beheizte Metalldrähte geführt wird (zweckmäßigerweise ein CSN Durchlauferhitzer, Typ 97D/80 der Fa. C. Schniewindt KG, 58805 Neuerade - DE).

Prinzipiell sieht die Drehrohrvorrichtung die Möglichkeit vor, den durch das Drehrohr geführten Gasstrom teilweise oder vollständig im Kreis zu führen. Die dazu erforderliche Kreisleitung wird am Drehrohreintritt und am Drehrohraustritt über Kugellager oder über Graphitpressdichtungen mit dem Drehrohr beweglich verbunden. Diese Verbindungen werden mit Inertgas (z.B. Stickstoff) gespült (Sperrgas). Die beiden Spülströme (11) ergänzen den durch das Drehrohr geführten Gasstrom am Eintritt ins Drehrohr und am Austritt aus dem Drehrohr. Zweckmäßigerweise verjüngt sich dabei das Drehrohr an seinem Anfang und an seinem Ende und ragt in das zu- bzw. wegführende Rohr der Kreisleitung hinein.

Hinter dem Austritt des durch das Drehrohr geführten Gasstroms befindet sich ein Zyklon (12), zur Abtrennung von mit dem Gasstrom mitgerissenen Feststoffpartikeln (der Zentrifugalabscheider trennt in der Gasphase suspendierte Feststoffpartikel durch Zusammenwirken von Zentrifugal- und Schwerkraft ab; die Zentrifugalkraft des als Spiralwirbel rotierenden Gasstroms beschleunigt die Sedimentation der suspendierten Teilchen).

Die Förderung des Kreisgasstroms (24) (die Gaszirkulation) erfolgt mittels eines Kreisgasverdichters (13) (Ventilators), der in Richtung des Zyklons ansaugt und in die andere Richtung drückt. Unmittelbar hinter dem Kreisgasverdichter liegt der Gasdruck in der Regel oberhalb einer Atmosphäre. Hinter dem Kreisgasverdichter befindet sich ein Kreisgasauslaß (über ein Regelventil (14) kann Kreisgas ausgeschleust werden). Eine hinter dem Auslass befindliche Blende (Querschnittsverjüngung um etwa einen Faktor 3, Druckminderer) (15) erleichtert den Auslass.

Über das Regelventil lässt sich der Druck hinter dem Drehrohrausgang regulieren. Dies geschieht im Zusammenspiel mit einem hinter dem Drehrohrausgang angebrachten Drucksensor (16), dem Abgasverdichter (17) (Ventilator), der zum Regelventil hin gerichtet ansaugt, dem Kreisgasverdichter (13) und der Frischgaszufuhr. Relativ zum Außendruck kann der Druck (unmittelbar) hinter dem Drehrohrausgang z.B. bis zu +1,0 mbar oberhalb und z.B. bis zu -1,2 mbar unterhalb liegend eingestellt werden. D.h., der Druck des das Drehrohr durchströmenden Gasstroms kann beim Verlassen des Drehrohrs unterhalb des Umgebungsdrucks des Drehrohrs liegen.

Ist beabsichtigt, den durch das Drehrohr geführten Gasstrom auch nicht wenigstens anteilig im Kreis zu führen, wird die Verbindung zwischen Zyklon (12) und Kreisgasverdichter (13) nach dem Dreiwegehahnprinzip (26) geschlossen und der Gasstrom auf direktem Weg in die Abgasreinigungsvorrichtung (23) geführt. Die hinter dem Kreisgasverdichter befindliche Verbindung zur Abgasreinigungsvorrichtung wird in diesem Fall ebenfalls nach dem Dreiwegehahnprinzip geschlossen. Besteht der Gasstrom im wesentlichen aus Luft, wird diese in diesem Fall über den Kreisgasverdichter (13) angesaugt (27). Die Verbindung zum Zyklon ist nach dem Dreiwegehahnprinzip geschlossen. Vorzugsweise wird der Gasstrom in diesem Fall durch das Drehrohr gesaugt, so dass der Drehrohrinnendruck kleiner als der Umgebungsdruck ist.

Bei kontinuierlichem Betrieb der Drehrohrofenvorrichtung wird der Druck hinter dem Drehrohrausgang vorteilhaft -0,2 mbar unterhalb des Außendruckes liegend eingestellt. Bei diskontinuierlichem Betrieb der Drehrohrvorrichtung wird der Druck hinter dem Drehrohrausgang vorteilhaft -0,8 mbar unterhalb des Außendruckes liegend eingestellt. Der leichte Unterdruck dient dem Zweck einer Vermeidung einer Kontamination der Umgebungsluft mit Gasgemisch aus dem Drehrohrofen.

Zwischen dem Kreisgasverdichter und dem Zyklon befinden sich Sensoren (18), die zum Beispiel den Ammoniakgehalt und den Sauerstoffgehalt im Kreisgas bestimmen. Der Ammoniaksensor arbeitet bevorzugt nach einem optischen Messprinzip (die Absorption von Licht bestimmter Wellenlänge korreliert proportional zum Ammoniakgehalt des Gases) und ist zweckmäßig ein Gerät der Fa. Perkin & Elmer vom Typ MCS 100. Der Sauerstoffsensor fußt auf den paramagnetischen Eigenschaften des Sauerstoffs und ist zweckmäßigerweise ein Oximat der Fa. Siemens vom Typ Oxymat MAT SF 7MB1010-2CA01-1AA1-Z.

Zwischen der Blende (15) und dem Erhitzer (10) können Gase wie Luft, Stickstoff, Ammoniak oder andere Gase zum tatsächlich rezirkulierten Kreisgasanteil (19) zudosiert werden. Häufig wird eine Grundlast an Stickstoff zudosiert (20). Mit einem separaten Stickstoff/Luft Splitter (21) kann auf den Messwert des Sauerstoffsensors reagiert werden.

Der ausgeschleuste Kreisgasanteil (22) (Abgas) enthält häufig nicht völlig unbedenkliche Gase wie NOₓ, Essigsäure, NH₃, u.s.w.), weshalb diese im Normalfall in einer Abgasreinigungsvorrichtung abgetrennt werden (23).

Dazu wird das Abgas in der Regel zunächst über eine Waschkolonne geführt (ist im wesentlichen eine an Einbauten freie Kolonne, die vor ihrem Ausgang eine trennwirksame Packung enthält; das Abgas und wässriger Sprühnebel werden im Gleichstrom und im Gegenstrom geführt (2 Sprühdüsen mit entgegengesetzter Sprührichtung)).

Aus der Waschkolonne kommend wird das Abgas in eine Vorrichtung geführt, die einen Feinstaubfilter (in der Regel ein Bündel aus Schlauchfiltern) enthält aus dessen Innerem das eingedrungene Abgas weggeführt wird. Dann wird abschließend in einer Muffel verbrannt.

Mittels eines Sensors (28) der Fa. KURZ Instruments, Inc., Montery (USA) vom Typ Modell 455 Jr wird die Menge des vom Sperrgas verschiedenen, dem Drehrohr zugeführten, Gasstroms gemessen und geregelt (Messprinzip: thermalkonvektive Massendurchflussmessung unter Anwendung eines Gleichtemperatur-Anemometers).

Bei kontinuierlichem Betrieb werden Materialgut und Gasphase im Gegenstrom durch den Drehrohrofen geführt.

Stickstoff bedeutet im Zusammenhang mit diesem Beispiel immer Stickstoff mit einer Reinheit > 99 Vol-%.

### 2. Herstellung der Ausgangsmasse 1 (Phase B) mit der Stöchiometrie Cu₁MO_{0,5}W_{0,5}O₄

Zu 603 I Wasser wurden 98 I einer 25 gew.-%igen wässrigen NH₃-Lösung gegeben. Anschließend wurden in der wässrigen Mischung 100 kg Kupfer(II)acetathydrat (Gehalt: 40,0 Gew.-% CuO) gelöst, wobei eine klare, tiefblaue wässrige Lösung 1 entstand, die 3,9 Gew.-% Cu enthielt und Raumtemperatur aufwies.

Unabhängig von der Lösung 1 wurden 620 I Wasser auf 40°C erwärmt. Darin wurden unter Aufrechterhaltung der 40°C 27,4 kg Ammoniumheptamolybdattetrahydrat (81,5 Gew.-% MoO₃) innerhalb von 20 min. unter Rühren gelöst. Dann wurden 40,4 kg Ammoniumparawolframatheptahydrat (88,9 Gew.-% WO₃) zugefahren und nach Aufheizen auf 90°C innerhalb von 45 min. bei dieser Temperatur unter Rühren gelöst. Es wurde eine klare, gelb-orange gefärbte wässrige Lösung 2 erhalten.

Anschließend wurde die wässrige Lösung 1 in die 90°C aufweisende Lösung 2 eingerührt, wobei die Temperatur der Gesamtmischung nicht unter 80°C sank. Die resultierende wässrige Suspension wurde 30 min. bei 80°C nachgerührt. Danach wurde sie mit einem Sprühtrockner der Fa. Niro-Atomizer (Kopenhagen) vom Typ S-50-N/R sprühgetrocknet (Gaseintrittstemperatur: 315°C, Gasaustrittstemperatur: 110°C, Gleichstrom). Das Sprühpulver wies einen Partikeldurchmesser von 2 bis 50 µm auf.

100 kg von so erhaltenem hellgrünem Sprühpulver wurden in einen Kneter der Fa. AMK (Aachener Misch- und Knetmaschinen Fabrik) vom Typ VIU 160 (Sigma Schaufeln) dosiert und unter Zugabe von 8 I Wasser geknetet (Verweilzeit: 30 min, Temperatur: 40 bis 50°C). Danach wurde das Knetgut in einen Extruder entleert und mittels des Extruders (Fa. Bonnot Company (Ohio), Typ: G 103-10/D7A-572K (6" Extruder W/Packer)) zu Strängen (Länge: 1-10 cm; Durchmesser: 6 mm) geformt. Auf einem Bandtrockner wurden die Stränge 1 h bei einer Temperatur von 120°C (Materialguttemperatur) getrocknet. Die getrockneten Stränglinge wurden anschließend im unter "1." beschriebenen Drehrohrofen wie folgt thermisch behandelt (calciniert):
- die thermisch Behandlung erfolgte kontinuierlich mit einem Materialguteintrag von 50 kg/h an Stränglingen;
- der Neigungswinkel des Drehrohres zur Horizontalen betrug 2°;
- im Gegenstrom zum Materialgut wurde ein Luftstrom von 75 Nm³/h durch das Drehrohr geführt, der von insgesamt (2 x 25) 50 Nm³/h Sperrgas der Temperatur 25°C ergänzt wurde;
- der Druck hinter dem Drehrohrausgang lag 0,8 mbar unter dem Außendruck;
- das Drehrohr rotierte mit 1,5 Umdrehungen/min links herum;
- es wurde keine Kreisgasfahrweise angewendet;
- beim ersten Durchgang der Stränglinge durch das Drehrohr wurde die Temperatur der Drehrohraußenwand auf 340°C eingestellt, der Luftstrom wurde mit einer Temperatur von 20 bis 30°C in das Drehrohr geführt;
- anschließend wurden die Stränglinge mit der selben Durchsatzmenge und abgesehen von den nachfolgenden Unterschieden unter den gleichen Bedingungen durch das Drehrohr geführt:
   - die Temperatur der Drehrohrwand wurde auf 790°C eingestellt;
   - der Luftstrom wurde auf eine Temperatur von 400°C erwärmt in das Drehrohr geführt.

Anschließend wurden die eine rotbraune Farbe aufweisenden Stränglinge auf einer Biplexquerstromsichtmühle (BQ 500) der Fa. Hosokawa-Alpine (Augsburg) auf einen mittleren Partikeldurchmesser von 3 bis 5 µm gemahlen. Die so erhaltene Ausgangsmasse 1 wies eine BET-Oberfläche ≤1 m²/g auf. Mittels Röntgenbeugung wurden folgende Phasen festgestellt:
1. CuMoO₄-III mit Wolframitstruktur;
2. HT-Kupfermolybdat.

### 3. Herstellung der Ausgangsmasse 2 (Phase C) mit der Stöchiometrie CuSb₂O₆

Unter Rühren wurden 52 kg Antimontrioxid (99,9 Gew.-% Sb₂O₃) in 216 I Wasser (25°C) suspendiert. Die resultierende wässrige Suspension wurde auf 80°C erwärmt. Anschließend wurde unter Aufrechterhaltung der 80°C 20 min nachgerührt. Anschließend wurden innerhalb einer Stunde 40 kg einer 30 gew.-%igen wässrigen Wasserstoffperoxidlösung zugegeben, wobei die 80°C aufrechterhalten wurden. Unter Beibehalt dieser Temperatur wurde 1,5 Stunden nachgerührt. Dann wurden 20 I 60°C warmes Wasser zugegeben und so eine wässrige Suspension 1 erhalten. In diese wurden bei einer Temperatur von 70°C 618,3 kg einer wässrigen ammoniakalischen Kupfer(II)acetatlösung (60,8 g Kupferacetat pro kg Lösung und 75 I einer 25 gew.-%igen wässrigen Ammoniaklösung in den 618,3 kg Lösung) eingerührt. Dann wurde auf 95°C erwärmt und bei dieser Temperatur 30 min nachgerührt. Dann wurden noch 50 I 70°C warmes Wasser ergänzt und auf 80°C erwärmt.

Schließlich wurde die wässrige Suspension sprühgetrocknet (Sprühtrockner der Fa. Niro-Atomizer (Kopenhagen) vom Typ S-5O-N/R, Gaseintrittstemperatur 360°C, Gasaustrittstemperatur 110°C, Gleichstrom). Das Sprühpulver wies einen Partikeldurchmesser von 2 bis 50 µm auf.

75 kg von so erhaltenem Sprühpulver wurden in einen Kneter der Fa. AMK (Aachener Misch- und Knetmaschinen Fabrik vom Typ VIU 160 (Sigma Schaufeln)) dosiert und unter Zugabe von 12 I Wasser geknetet (Verweilzeit: 30 min, Temperatur 40 bis 50°C). Danach wurde das Knetgut in einen Extruder (gleicher Extruder wie bei Phase B Herstellung) entleert und mittels des Extruders zu Strängen (Länge 1-10 cm; Durchmesser 6 mm) geformt. Auf einem Bandtrockner wurden die Stränge 1 h bei einer Temperatur von 120°C (Materialguttemperatur) getrocknet.

250 kg von so erhaltenen Strängen wurden im unter "1." beschriebenen Drehrohrofen wie folgt thermisch behandelt (calciniert):
- die thermische Behandlung erfolgte diskontinuierlich mit einer Materialgutmenge von 250 kg;
- der Neigungswinkel des Drehrohres zur Horizontalen betrug ≈0°;
- das Drehrohr rotierte mit 1,5 Umdrehungen/min rechts herum;
- durch das Drehrohr wurde ein Gasstrom von 205 Nm³/h geführt; dieser bestand zu Beginn der thermischen Behandlung aus 180 Nm³/h Luft und 1 x 25 Nm³/h N₂ als Sperrgas; der das Drehrohr verlassende Gasstrom wurde um weitere 1 x 25 Nm³/h N₂ ergänzt; von diesem Gesamtstrom wurden 22 - 25 Vol.% ins Drehrohr rückgeführt und der Rest ausgelassen; die Auslassmenge wurde durch das Sperrgas und als Restmenge durch Frischluft ergänzt;
- der Gasstrom wurde mit 25°C ins Drehrohr geführt;
- der Druck hinter dem Drehrohrausgangs lag 0,5 mbar unterhalb Außendruck (Normaldruck);
- die Temperatur im Materialgut wurde zunächst innerhalb von 1,5 h linear von 25°C auf 250°C erhöht; dann wurde die Temperatur im Materialgut innerhalb von 2 h linear von 250°C auf 300°C erhöht und diese Temperatur 2 h gehalten; dann wurde die Temperatur im Materialgut innerhalb von 3 h linear von 300°C auf 405°C erhöht und diese Temperatur anschließend 2 h gehalten; dann wurden die Heizzonen abgeschaltet und die Temperatur innerhalb des Materialgutes durch Aktivierung der Schnellkühlung des Drehrohres durch Ansaugen von Luft innerhalb von 1 h auf eine unterhalb von 100°C liegende Temperatur erniedrigt und schließlich auf Umgebungstemperatur abgekühlt.

Die so resultierende pulverförmige Ausgangsmasse 2 wies eine spezifische BET-Oberfläche von 0,6 m²/g und die Zusammensetzung CuSb₂O₆ auf. Das Pulver-Röntgendiagramm des erhaltenen Pulvers zeigte im wesentlichen die Beugungsreflexe von CuSb₂O₆ (Vergleichsspektrum 17-0284 der JCPDS-ICDD-Kartei).

### 4. Herstellung der Ausgangsmasse 3 mit der Stöchiometrie MO₁₂V_{3,35}W_{1,38}

In einem Rührkessel wurden bei 25°C unter Rühren 900 I Wasser vorgelegt. Anschließend wurden 122,4 kg Ammoniumheptamolybdattetrahydrat (81,5 Gew.-% MoO₃) zugegeben und unter Rühren auf 90°C erhitzt. Dann wurden unter Aufrechterhaltung der 90°C zunächst 22,7 kg Ammoniummetavanadat und schließlich 20,0 kg Ammoniumparawolframatheptahydrat (88,9 Gew.-% WO₃) eingerührt. Durch insgesamt 80-minütiges Rühren bei 90°C wurde eine klare orangefarbene Lösung erhalten. Diese wurde auf 80°C abgekühlt. Dann wurden unter Beibehalt der 80°C zunächst 18,8 kg Essigsäure (=100 gew.-%ig, Eisessig) und dann 24 I 25 gew.-%ige wässrige Ammoniaklösung eingerührt.

Die Lösung blieb klar und orangefarben und wurde mit einem Sprühtrockner der Fa. Niro-Atomizer (Kopenhagen) vom Typ S-50-N/R sprühgetrocknet (Gaseintrittstemperatur: 260°C, Gasaustrittstemperatur: 110°C, Gleichstrom). Das resultierende Sprühpulver bildete die Ausgangsmasse 3 und wies einen Partikeldurchmesser von 2 bis 50µm auf.

### 5. Herstellung der erfindungsgemäß thermisch zu behandelnden Trockenmasse mit der Stöchiometrie (MO₁₂V_{3,46}W_{1,39})_{0,87} (CuMo_{0,5}W_{0,5}O₄)_{0,4} (CuSb₂O₆)_{0,4}

In einem Trogkneter der Fa. AMK (Aachener Misch- und Knetmaschinen Fabrik) vom Typ VIU 160 mit zwei Sigmaschaufeln wurden 75 kg Ausgangsmasse 3, 5,2 1 Wasser und 6,9 kg Essigsäure (100 Gew.-% Eisessig) vorgelegt und während 22 min geknetet. Anschließend wurden 3,1 kg Ausgangsmasse 1 und 4,7 kg Ausgangsmasse 2 zugegeben und während weiterer 8 min geknetet (T ≈40 bis 50°C).

Danach wurde das Knetgut in einen Extruder (gleicher Extruder wie bei Phase B Herstellung) entleert und mittels des Extruders zu Strängen (1 bis 10 cm Länge, 6 mm Durchmesser) geformt. Diese wurden auf einem Bandtrockner während 1 h bei einer Temperatur (Materialguttemperatur) von 120°C getrocknet.

306 kg der getrockneten Stränglinge wurden anschließend im unter "1." beschriebenen Drehrohrofen wie folgt thermisch behandelt:
- die thermische Behandlung erfolgte diskontinuierlich mit einer Materialgutmenge von 306 kg;
- der Neigungswinkel des Drehrohres zur Horizontalen betrug ≈0°;
- das Drehrohr rotierte mit 1,5 Umdrehungen/min rechts herum;
- zunächst wurde die Materialguttemperatur innerhalb von 2 h im wesentlichen linear von 25°C auf 100°C erhitzt;
   während dieser Zeit wird durch das Drehrohr ein (im wesentlichen) Stickstoffstrom von 205 Nm³/h zugeführt. Im stationären Zustand (nach Verdrängung der ursprünglich enthaltenen Luft) ist dieser wie folgt zusammengesetzt:
   110 Nm³/h Grundlast - Stickstoff (20),
   25 Nm³/h Sperrgas - Stickstoff (11), und
   70 Nm³/h rezirkuliertes Kreisgas (19).

Der Sperrgas-Stickstoff wurde mit einer Temperatur von 25°C zugeführt. Das Gemisch der beiden anderen Stickstoffströme wurde jeweils mit der Temperatur ins Drehrohr geführt, die das Materialgut im Drehrohr jeweils aufwies.
- anschießend wurde die Materialguttemperatur mit einer Heizrate von 0,7°C/min von 100°C auf 320°C aufgeheizt;
bis zum Erreichen einer Materialguttemperatur von 300°C wurde durch das Drehrohr ein Gasstrom von 205 Nm³/h geführt, der wie folgt zusammengesetzt war:
110 Nm³/h bestehend aus Grundlast - Stickstoff (20) und im Drehrohr freigesetzten Gasen,
25 Nm³/h Sperrgas - Stickstoff (11) und
70 Nm³/h rezirkuliertes Kreisgas (19).

Der Sperrgas-Stickstoff wurde mit einer Temperatur von 25°C zugeführt. Das Gemisch der beiden anderen Gasströme wurde jeweils mit der Temperatur ins Drehrohr geführt, die das Materialgut im Drehrohr jeweils aufwies.

Ab Überschreiten der Materialguttemperatur von 160°C bis zum Erreichen einer Materialguttemperatur von 300°C wurden aus dem Materialgut 40 mol-% der im Verlauf der gesamten thermischen Behandlung des Materialgutes insgesamt freigesetzten Ammoniakmenge M^{A} freigesetzt.
- bei Erreichen der Materialguttemperatur von 320°C wurde der Sauerstoffgehalt des dem Drehrohr zugeführten Gasstromes von 0 Vol.-% auf 1,5 Vol.-% erhöht und über die nachfolgenden 4 h beibehalten.

Gleichzeitig wurde die in den vier das Drehrohr beheizenden Heizzonen herrschende Temperatur um 5°C abgesenkt (auf 325°C) und so während der nachfolgenden 4 h aufrechterhalten.

Die Materialguttemperatur durchlief dabei ein oberhalb von 325°C liegendes Temperaturmaximum, das 340°C nicht überschritt, bevor die Materialguttemperatur wieder auf 325°C sank.

Die Zusammensetzung des durch das Drehrohr geführten Gasstroms von 205 Nm³/h wurde während dieses Zeitraums von 4 h wie folgt geändert:
95 Nm³/h bestehend aus Grundlast - Stickstoff (20) und im Drehrohr freigesetzten Gasen;
25 Nm³/h Sperrgas - Stickstoff (11);
70 Nm³/h rezirkuliertes Kreisgas; und
15 Nm³/h Luft über den Splitter (21).

Der Sperrgas-Stickstoff wurde mit einer Temperatur von 25°C zugeführt.

Das Gemisch der anderen Gasströme wurde jeweils mit der Temperatur ins Drehrohr geführt, die das Materialgut im Drehrohr jeweils aufwies.

Ab Überschreiten der Materialguttemperatur von 300°C bis zum Ablauf der 4 h wurden aus dem Materialgut 55 mol-% der im Verlauf der gesamten thermischen Behandlung des Materialguts insgesamt freigesetzten Ammoniakmenge M^{A} freigesetzt (damit waren bis zum Ablauf der 4 h insgesamt 40 mol-% + 55 mol-% = 95 mol-% der Ammoniakmenge M^{A} freigesetzt).
- mit Ablauf der4 h wurde die Temperatur des Materialguts mit einer Heizrate von 0,85°C/min innerhalb von etwa 1,5 h auf 400°C erhöht.

Anschließend wurde diese Temperatur 30 min aufrechtgehalten.

Die Zusammensetzung des dem Drehrohr zugeführten Gasstroms von 205 Nm³/h war während dieser Zeit wie folgt:
95 Nm³/h zusammengesetzt aus Grundlast - Stickstoff (20) und im Drehrohr freigesetzten Gasen;
15 Nm³/h Luft (Splitter (21));
25 Nm³/h Sperrgas-Stickstoff (11); und
70 Nm³/h rezirkuliertes Kreisgas.

Der Sperrgas-Stickstoff wurde mit einer Temperatur von 25°C zugeführt. Das Gemisch der anderen Gasströme wurde jeweils mit der Temperatur ins Drehrohr geführt, die das Materialgut im Drehrohr jeweils aufwies.
- durch Verringerung der Temperatur des Materialguts wurde die Calcination beendet; dazu wurden die Heizzonen aus- und die Schnellkühlung des Drehrohres durch Ansaugen von Luft eingeschaltet, und die Materialguttemperatur innerhalb von 2 h auf eine unterhalb von 100°C liegende Temperatur erniedrigt und schließlich auf Umgebungstemperatur abgekühlt;
mit der Abschaltung der Heizzonen wurde die Zusammensetzung des dem Drehrohr zugeführten Gasstroms von 205 Nm³/h auf folgendes Gemisch verändert:
110 Nm³/h zusammengesetzt aus Grundlast - Stickstoff (20) und im Drehrohr freigesetzten Gasen;
0 Nm³/h Luft (Splitter (21));
25 Nm³/h Sperrgas-Stickstoff (11); und
70 Nm³/h rezirkuliertes Kreisgas.

Der Gasstrom wurde dem Drehrohr mit einer Temperatur von 25°C zugeführt.
- während der gesamten thermischen Behandlung lag der Druck (unmittelbar) hinter dem Drehrohrausgang 0,2 mbar unterhalb des Außendrucks.

### 6. Formgebung der Multimetalloxidaktivmasse

Das in "5." erhaltene katalytisch aktive Material wurde mittels einer Biplexquerstromsichtmühle (BQ 500) (Fa. Hosokawa-Alpine Augsburg) zu einem feinteiligen Pulver gemahlen, von dem 50 % der Pulverpartikel ein Sieb der Maschenweite 1 bis 10 µm passierten und dessen Anteil an Partikeln mit einer Längstausdehnung oberhalb von 50µm weniger als 1 % betrug. Die spezifische Oberfläche des Multimetalloxidaktivmassenpulvers betrug .... cm²/g.

Mittels des gemahlenen Pulvers wurden wie in S1 der EP-B 714700 ringförmige Trägerkörper (7 mm Außendurchmesser, 3 mm Länge, 4 mm Innendurchmesser, Steatit des Typs C220 der Fa. CeramTec mit einer Oberflächenrauhigkeit R_{z} von 45µm) beschichtet. Bindemittel war eine wässrige Lösung aus 75 Gew.-% Wasser und 25 Gew.-% Glycerin.

Der Aktivmassenanteil der resultierenden Schalenkatalysatoren wurde jedoch im Unterschied zu vorgenanntem Beispiel S1 zu 20 Gew.-% (bezogen auf das Gesamtgewicht aus Trägerkörper und Aktivmasse) gewählt. Das Verhältnis von Pulver und Bindemittel wurde proportional angepasst.

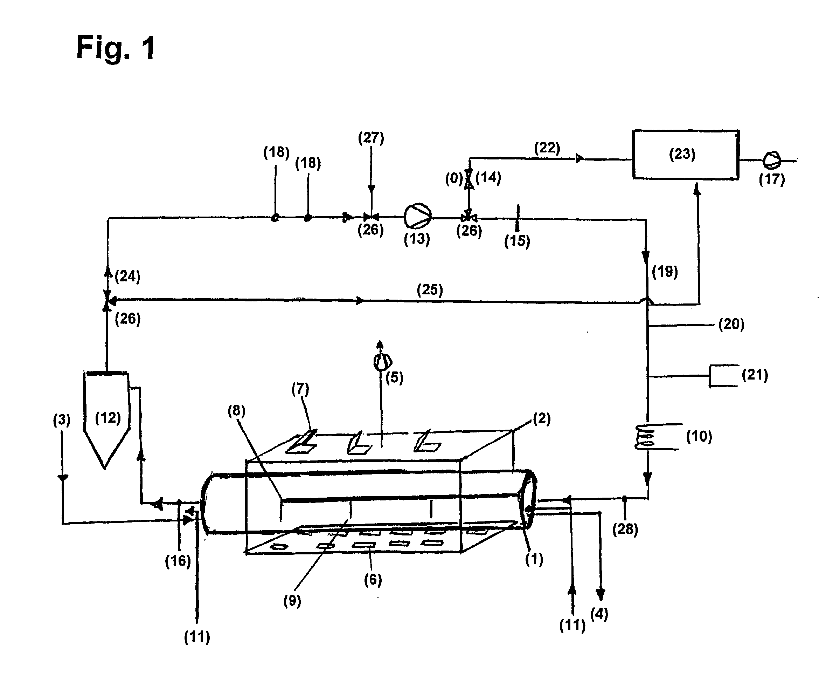

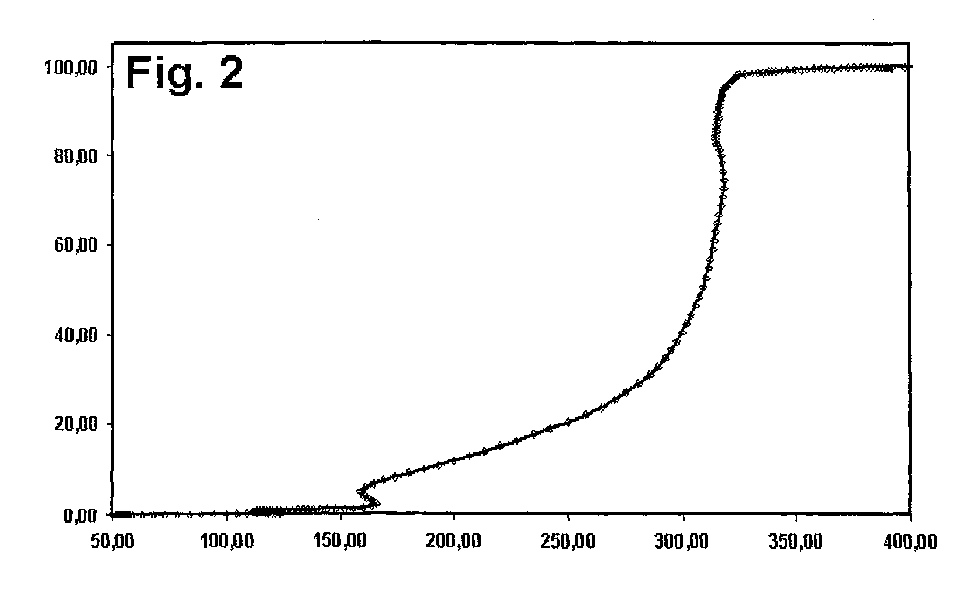

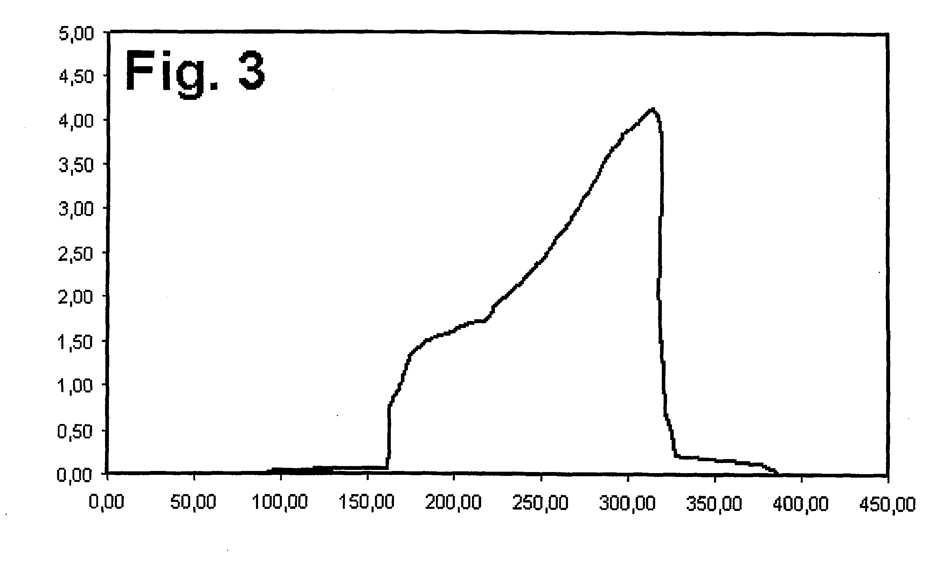

Figur 2 zeigt den prozentualen Anteil von M^{A} als Funktion der Materialguttemperatur in °C. Figur 3 zeigt die Ammoniakkonzentration der Atmosphäre A in Vol.-% über die thermische Behandlung in Abhängigkeit von der Materialguttemperatur in °C.

### 7. Testung der Schalenkatalysatoren

Die Schalenkatalysatoren wurden wie folgt in einem von einem Salzbad (Gemisch aus Kaliumnitrat und Natriumnitrat) umspülten Modellkontaktrohr getestet:
Modellkontaktrohr: V2A-Stahl, 2 mm Wandstärke, 26 mm Innendurchmesser, zentriert eine Thermohülse (zur Aufnahme eines Thermoelements) des Außendurchmessers 4 mm, 1,56 1 des freien Modellkontaktrohrraumes wurden mit dem Schalenkatalysator gefüllt;

Das Reaktionsgasgemisch wies folgende Ausgangszusammensetzung auf:
4,8 Vol.-% Acrolein, 7 Vol.% Sauerstoff, 10 Vol.% Wasserdampf, 76 Vol.% Stickstoff und als Restmenge ein Gemisch aus Kohlenoxiden und Oxygenaten des Propylens.
Das Modellkontaktrohr wurde mit 2800 NI/h an Reaktionsgasausgangsgemisch belastet. Die Temperatur des Salzbades wurde so eingestellt, dass bei einfachem Durchgang ein Acroleinumsatz von 99,3 mol-% resultierte.
Die diesbezüglich erforderliche Salzbadtemperatur T betrug 262°C und die Selektivität der Acrylsäurebildung lag bei 96,4 mol% Acrylsäure.

An dieser Stelle sei noch festgehalten, dass mit diesen Schalenkatalysatoren eine regelmäßige Beschickung von Rohrbündelreaktoren mit 5000 bis 40000 Kontaktrohren (Wanddicke typisch 1 bis 3 mm, Innendurchmesser in der Regel 20 bis 30 mm, häufig 21 bis 26 mm, Länge typisch 2 bis 4 m) möglich ist, die so beschaffen ist, dass infolge der homogenen Herstellung der Aktivmasse bei einer willkürlich herausgegriffenen Stichprobe von 12 Kontaktrohren der Unterschied zwischen der arithmetisch mittleren Aktivität und der höchsten bzw. kleinsten Aktivität nicht mehr als 8°C, häufig nicht mehr als 6°C, vielfach nicht mehr als 4°C und in günstigen Fällen nicht mehr als 2°C beträgt.

Als Maß für die Aktivität der Kontaktrohrbeschickung wird dabei die Temperatur verwendet, die ein das einzelne Kontaktrohr umspülende Salzbad (Gemisch aus 53 Gew.-% Kaliumnitrat, 40 Gew.-% Natriumnitrit und 7 Gew.-% Natriumnitrat) aufweisen muß, damit bei einmaligem Durchgang eines Reaktionsgasgemisches aus 4,8 Vol.% Acrolein, 7 Vol.% Sauerstoff, 10 Vol.% Wasserdampf und 78,2 Vol.-% Stickstoff (bei einer Belastung der Katalysatorbeschickung mit 85 NI Acrolein/I Katalysatorbeschickung - h) durch das beschickte Katalysatorrohr ein Acroleinumsatz von 97 mol% erzielt wird (unter "I Katalysatorbeschickung" werden dabei die Volumina innerhalb des Katalysatorrohres nicht mit einbezogen, wo sich reine Vor- oder Nachschüttungen aus Inertmaterial befinden, sondern nur die Schüttvolumina, die Katalysatorformkörper (gegebenenfalls mit Inertmaterial verdünnt) enthalten.

Solche Katalysatorbeschickungen eignen sich insbesondere für hohe Acroleinbelastungen (z.B. ≥135 NI/I•h bis 350 NI/I•h und mehr).

### Vergleichsbeispiel 1

Alles wurde wie im Beispiel 1 durchgeführt. Allerdings enthielt der dem Drehrohr zugeführte Gasgemischstrom von Anfang an (d.h., bereits auf dem Weg zur Materialguttemperatur von 100°C bzw. 300°C) 95 Nm³/h zusammengesetzt aus Grundlast-Stickstoff (20) und im Drehrohr freigesetzten Gasen und 70 Nm³/h rezirkuliertes Kreisgas, dafür jedoch von Anfang an 15 Nm³/h Luft (Splitter (21)) und damit 1,5 Vol.% molekularen Sauerstoff.

Die bei der Schalenkatalysatortestung für einen Acroleinumsatz von 99,3 mol-% erforderliche Salzbadtemperatur betrug 268°C und die Selektivität der Acrylsäurebildung lag nur bei 94,2 mol%.

### Vergleichsbeispiel 2

Alles wurde wie im Beispiel 1 durchgeführt. Während der Gesamtdauer der thermischen Behandlung enthielt der dem Drehrohr zugeführte Gasstrom jedoch keinerlei Luft (anstelle des Lufststroms gemäß Beispiel wurde über den Splitter immer ein entsprechender Stickstoffstrom zugeführt.

Die bei der Schalenkatalysatortestung für einen Acroleinumsatz von 99,3 mol-% erforderliche Salzbadtemperatur betrug 279°C und die Selektivität der Acrylsäurebildung lag nur bei 91,0 mol-%.

### Beispiel 2

Alles wurde wie im Beispiel 1 durchgeführt. Die Formgebung der Multimetalloxidaktivmasse erfolgte allerdings wie folgt:

70 kg ringförmige Trägerkörper (7,1 mm Außendurchmesser, 3,2 mm Länge, 4,0 mm Innendurchmesser; Steatit des Typs C220 der Fa. CeramTec mit einer Oberflächenrauhigkeit R_{z} von 45µm und einem auf das Volumen des Trägerkörpers bezogenen Porengesamtvolumen ≤1 Vol.-%) wurden in einen Dragierkessel (Neigungswinkel 90°; Hicoater der Fa: Lödige, DE) von 200 1 Innenvolumen gefüllt. Anschließend wurde der Dragierkessel mit 16 U/min in Rotation versetzt. Über eine Düse wurden innerhalb von 25 min 3,8 bis 4,2 Liter einer wässrigen Lösung aus 75 Gew.-% Wasser und 25 Gew.-% Glycerin auf die Trägerkörper aufgesprüht. Gleichzeitig wurden im selben Zeitraum 18,1 kg der gemahlenen Multimetalloxidaktivmasse über eine Schüttelrinne außerhalb des Sprühkegels der Zerstäuberdüse kontinuierlich zudosiert. Während der Beschichtung wurde das zugeführte Pulver vollständig auf die Oberfläche des Trägerkörpers aufgenommen, eine Agglomeration der feinteiligen oxidischen Aktivmasse wurde nicht beobachtet. Nach beendeter Zugabe von Aktivmassenpulver und Wasser wurde bei einer Drehgeschwindigkeit von 2 U/min 40 min (alternativ 15 bis 60 min) 100°C (alternativ 80 bis 120°C) heiße Luft (ca. 400 m³/h) in den Dragierkessel geblasen. Es wurden ringförmige Schalenkatalysatoren erhalten, deren Anteil an oxidischer Aktivmasse, bezogen auf die Gesamtmasse, 20 Gew.-% betrug. Die Schalendicke lag, sowohl über die Oberfläche eines Trägerkörpers als auch über die Oberfläche verschiedener Trägerkörper betrachtet, bei 170 ± 50 µm.

Die Testung der Schalenkatalysatoren erfolgte wie im Beispiel 1. Die resultierenden Ergebnisse entsprachen den in Beispiel 1 erzielten Resultaten.

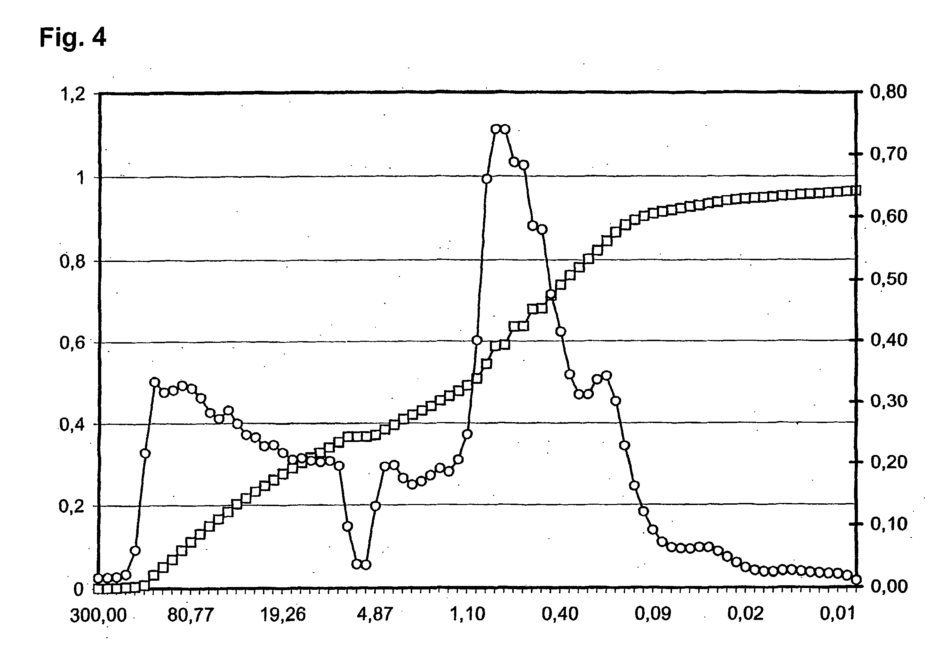

Figur 4 zeigt außerdem die Porenverteilung des gemahlenen Aktivmassenpulvers vor seiner Formgebung (seine spezifische Oberfläche betrug 21 m²/g). Auf der Abszisse ist der Porendurchmesser in µm aufgetragen (logarithmische Skala).

Auf der rechten Ordinate ist der Logarithmus des differentiellen Beitrags in ml/g des jeweilige Porendurchmessers zum Porengesamtvolumen aufgetragen (Kurve O). Das Maximum weist den Porendurchmesser mit dem größten Beitrag zum Porengesamtvolumen aus. Auf der linken Ordinate ist in ml/g das Integral über die individuellen Beiträge der einzelnen Porendurchmesser zum Porengesamtvolumen aufgetragen (Kurve □). Der Endpunkt ist das Porengesamtvolumen (alle Angaben in dieser Schrift zu Bestimmungen von Porengesamtvolumina sowie von Durchmesserverteilungen auf diese Porengesamtvolumina beziehen sich, soweit nichts anderes erwähnt wird, auf Bestimmungen mit der Methode der Quecksilberporosimetrie in Anwendung des Gerätes Auto Pore 9220 der Fa. Micromeritics GmbH, 4040 Neuß, DE (Bandbreite 30 Ä bis 0,3 mm); alle Angaben in dieser Schrift zu Bestimmungen von spezifischen Oberflächen bzw. von Mikroporenvolumina beziehen sich auf Bestimmungen nach DIN 66131 (Bestimmung der spezifischen Oberfläche von Feststoffen durch Gasadsorption (N₂) nach Brunauer-Emmet-Teller (BET))).

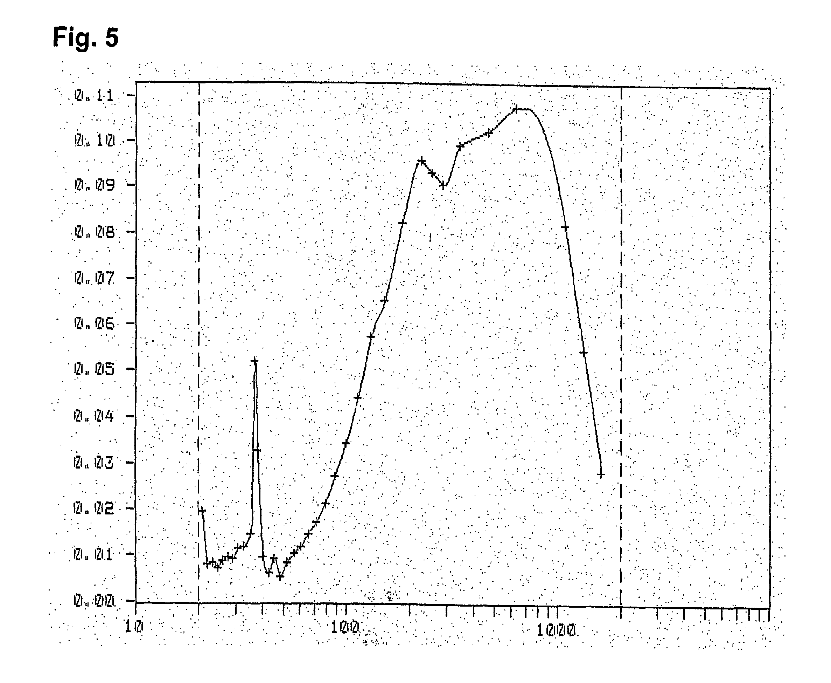

Figur 5 zeigt für das Aktivmassenpulver vor seiner Formgebung in ml/g (Ordinate) die individuellen Beiträge der einzelnen Porendurchmesser (Abszisse, in Angström, logarithmische Skala) im Mikroporenbereich zum Porengesamtvolumen.

Figur 6 zeigt das gleiche wie Figur 4, jedoch für vom ringförmigen Schalenkatalysator durch mechanisches Abkratzen nachträglich gelöste Multimetalloxidaktivmasse (ihre spezifische Oberfläche betrug 24,8 m²/g).

Figur 7 zeigt das gleiche wie Figur 5, jedoch für vom ringförmigen Schalenkatalysator durch mechanisches Abkratzen nachträglich gelöste Multimetalloxidaktivmasse.

### Beispiel 3

Alles wurde wie im Beispiel 1 durchgeführt. Die Formgebung der Multimetalloxidaktivmasse erfolgte allerdings wie folgt:

70 kg kugelförmige Trägerkörper (Durchmesser 4 bis 5 mm; Steatit des Typs C220 der Fa. CeramTec mit einer Oberflächenrauhigkeit R_{z} von 45µm und einem auf das Volumen des Trägerkörpers bezogenen Porengesamtvolumen ≤1 Vol.-%) wurde in einen Dragierkessel (Neigungswinkel 90°; Hicoater der Fa. Lödige, DE) von 200 1 Innenvolumen gefüllt. Anschließend wurde der Dragierkessel mit 16 U/min in Rotation versetzt. Über eine Düse wurden innerhalb von 25 min 2, 8 bis 3,3 Liter Wasser auf die Träger körper aufgesprüht. Gleichzeitig wurden im selben Zeitraum 14,8 kg der gemahlenen Multimetalloxidaktivmasse über eine Schüttelrinne außerhalb des Sprühkegels der Zerstäuberdüse kontinuierlich zudosiert. Während der Beschichtung wurde das zugeführte Pulver vollständig auf die Oberfläche der Trägerkörper aufgenommen, eine Agglomeration der feinteiligen oxidischen Aktivmasse wurde nicht beobachtet. Nach beendeter Zugabe von Pulver und Wasser wurde bei einer Drehgeschwindigkeit von 2 U/min 40 min (alternativ 15 bis 60 min) 100°C (alternativ 80 bis 120°C) heiße Luft (ca. 400 m³/h) in den Dreagierkessel geblasen. Es wurden kugelförmige Schalenkatalysatoren erhalten, deren Anteil an oxidischer Aktivmasse, bezogen auf die Gesamtmasse, 17 Gew.-% betrug. Die Schalendicke lag, sowohl über die Oberfläche eines Trägerkörpers als auch über die Oberfläche verschiedener Trägerkörper betrachtet, bei 160 ± 50µm.

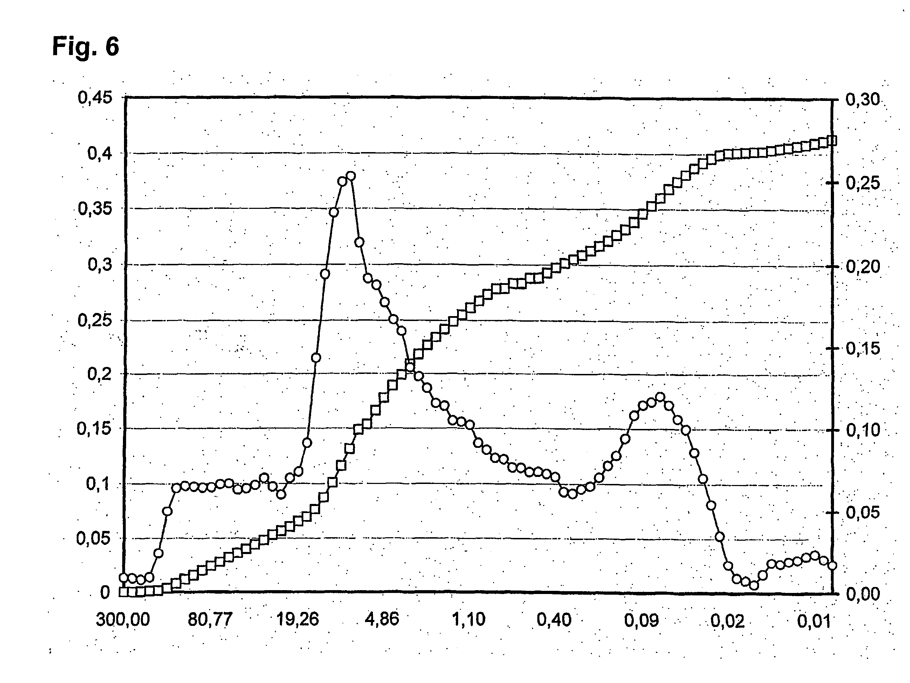

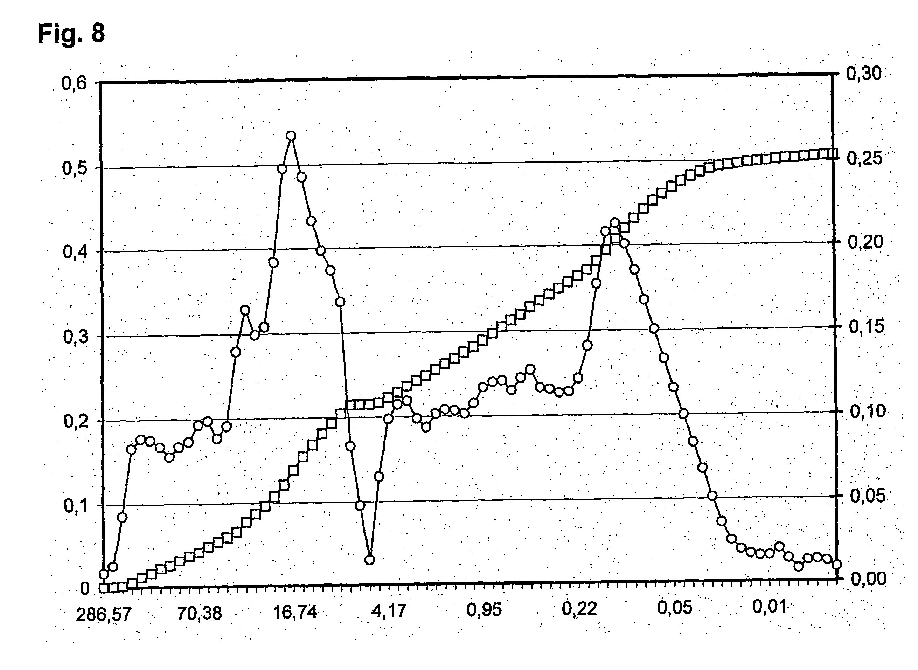

Figur 8 zeigt das Analogon zu Figur 6 (die spezifische Oberfläche der abgekratzten Multimetalloxidaktivmasse betrug 20,3 m²/g).

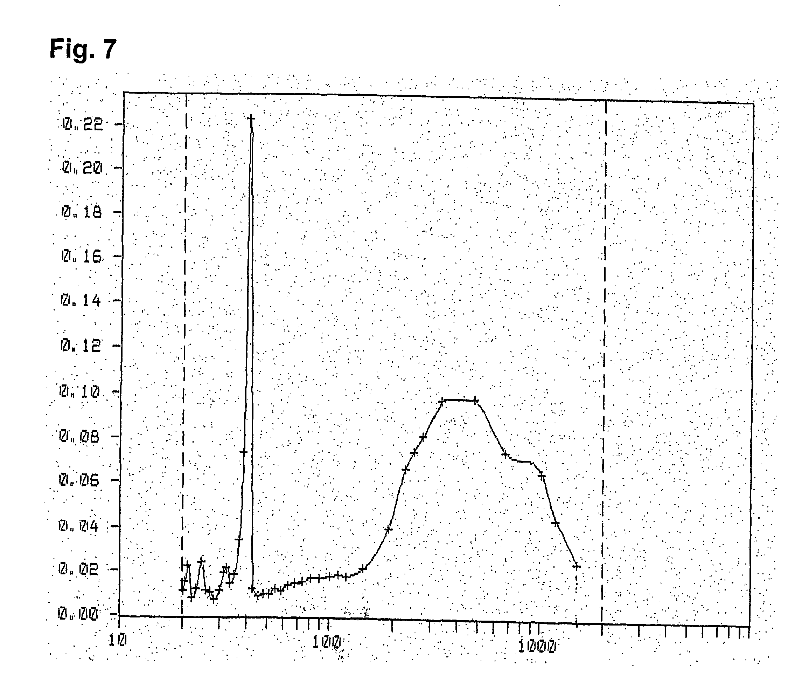

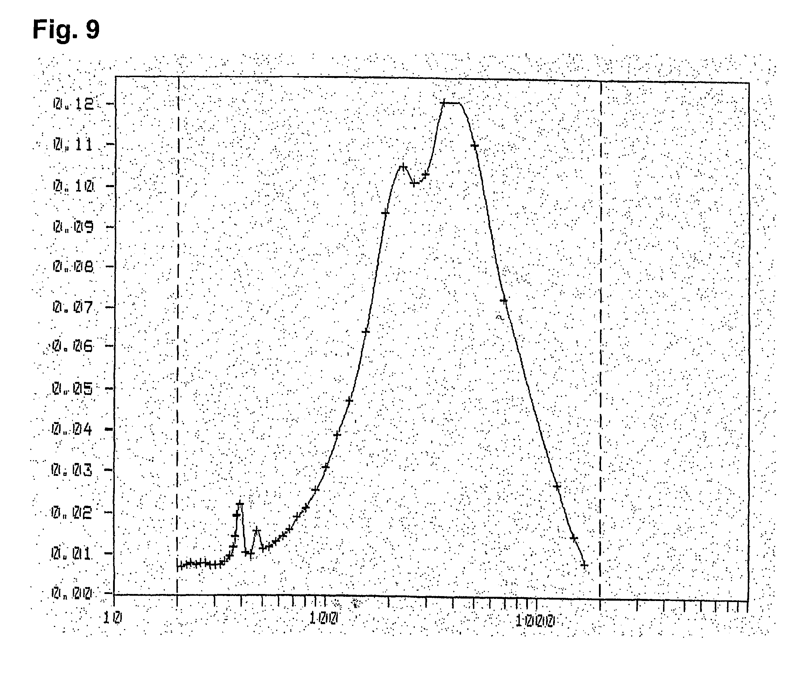

Figur 9 zeigt das Analogon zu Figur 7.

Die Testung des kugelförmigen Schalenkatalysators erfolgte wie in Beispiel 1 für den ringförmigen Schalenkatalysator beschrieben.

Alle in dieser Schrift beispielhaft hergestellten Schalenkatalysatoren eignen sich insbesondere für Acroleinpartialoxidationen bei hohen Acroleinbelastungen der Katalysatorbeschickung (z.B. ≥135 NI/I·h bis 350 NI/I•h).

## Patentansprüche

1. Verfahren zur Herstellung von katalytisch aktiven Multielementoxidmassen, die wenigstens eines der Elemente Nb und W sowie die Elemente Mo, V und Cu enthalten, wobei der molare Anteil des Elementes Mo an der Gesamtmenge aller von Sauerstoff verschiedenen Elemente der katalytisch aktiven Multielementoxidmasse 20 mol-% bis 80 mol% beträgt, das molare Verhältnis von in der katalytisch aktiven Multielementoxidmasse enthaltenem Mo zu in der katalytisch aktiven Multielementoxidmasse enthaltenem V, Mo/V, 15:1 bis 1:1 beträgt, das entsprechende molare Verhältnis Mo/Cu 30:1 bis 1:3 und das entsprechende molare Verhältnis Mo/(Gesamtmenge aus W und Nb) 80:1 bis 1:4 beträgt und bei dem man aus Ausgangsverbindungen, die die von Sauerstoff verschiedenen elementaren Konstituenten der Multielementoxidmasse als Bestandteile enthalten, ein inniges, auch Ammoniumionen enthaltendes, Trockengemisch herstellt und dieses in einer an molekularem Sauerstoff armen Atmosphäre bei erhöhter Temperatur thermisch behandelt, wobei bei Temperaturen ≥160°C wenigstens eine Teilmenge der im innigen Trockengemisch enthaltenen Ammoniumionen unter Freisetzung von Ammoniak zersetzt wird, **dadurch gekennzeichnet, dass** die thermische Behandlung wie folgt erfolgt:
- das innige Trockengemisch wird mit einer Temperaturrate von ≤10°C/min auf eine Zersetzungstemperatur im Zersetzungstemperaturbereich von 240°C bis 360°C erwärmt und so lange in diesem Temperaturbereich gehalten, bis wenigstens 90 mol-% der im Gesamtverlauf der thermischen Behandlung des innigen Trockengemischs aus dem innigen Trockengemisch bei Temperaturen oberhalb von 160°C insgesamt freigesetzten Gesamtmenge M^{A} an Ammoniak freigesetzt worden sind;
- spätestens dann, wenn das innige Trockengemisch die Temperatur von 230°C erreicht hat, wird der Gehalt der Atmosphäre A, in der sich die thermische Behandlung des innigen Trockengemischs vollzieht, an molekularem Sauerstoff auf einen Wert von ≤0,5 Vol.% abgesenkt und dieser niedere Sauerstoffgehalt so lange aufrechterhalten, bis wenigstens 20 mol-% der im Gesamtverlauf der thermischen Behandlung insgesamt freigesetzten Gesamtmenge M^{A} an Ammoniak freigesetzt worden sind;
- frühestens dann, wenn ≥ 70 mol-% der im Gesamtverlauf der thermischen Behandlung insgesamt freigesetzten Gesamtmenge M^{A} an Ammoniak freigesetzt worden sind, wird das innige Trockengemisch mit einer Rate von ≤10°C/min aus dem Zersetzungstemperaturbereich heraus - und in den Calcinationstemperaturbereich von 380 bis 450°C hineingeführt und
- spätestens dann, wenn 98 mol-% der im Gesamtverlauf der thermischen Behandlung insgesamt freigesetzten Gesamtmenge M^{A} an Ammoniak freigesetzt worden sind, wird der Gehalt der Atmosphäre A an molekularem Sauerstoff auf > 0,5 Vol.% bis 4 Vol.% erhöht
und das innige Trockengemisch bei diesem erhöhten Sauerstoffgehalt der Atmosphäre A im Calcinationstemperaturbereich calciniert.

2. Verfahren nach Anspruch 1, **dadurch gekennzeichnet, dass** der Gehalt des ther misch zu behandelnden innigen Trockengemischs an Ammoniumionen, bezogen auf den molaren Gesamtgehalt des innigen Trockengemischs an von Sauerstoff verschiedenen elementaren Konstituenten der späteren katalytisch aktiven Multielementoxidmasse, ≥5 mol-% beträgt.

3. Verfahren nach Anspruch 1 oder 2, **dadurch gekennzeichnet, dass** die Temperaturrate, mit der das innige Trockengemisch auf die Zersetzungstemperatur erwärmt wird, ≤5°C/min beträgt.

4. Verfahren nach einem der Ansprüche 1 bis 3, **dadurch gekennzeichnet, dass** die Temperaturrate, mit der das innige Trockengemisch auf die Zersetzungstemperatur erwärmt wird, ≤3°C/min beträgt.

5. Verfahren nach einem der Ansprüche 1 bis 4, **dadurch gekennzeichnet, dass** sich der Zersetzungstemperaturbereich auf den Bereich von 300 bis 350°C erstreckt.

6. Verfahren nach einem der Ansprüche 1 bis 5, **dadurch gekennzeichnet, dass** das innige Trockengemisch so lange im Zersetzungstemperaturbereich gehalten wird, bis wenigstens 95 mol-% der im Gesamtverlauf der thermischen Behandlung des innigen Trockengemischs aus dem innigen Trockengemisch bei Temperaturen oberhalb von 160°C insgesamt freigesetzten Gesamtmenge M^{A} an Ammoniak freigesetzt worden sind.

7. Verfahren nach einem der Ansprüche 1 bis 6, **dadurch gekennzeichnet, dass** spätestens dann, wenn das innige Trockengemisch die Temperatur von 230°C erreicht hat, der Gehalt der Atmosphäre A an molekularem Sauerstoff ≤0,3 Vol.-% beträgt.

8. Verfahren nach einem der Ansprüche 1 bis 7, **dadurch gekennzeichnet, dass** der Gehalt der Atmosphäre A an molekularem Sauerstoff auf einen Wert > 0,5 Vol.% bis 4 Vol.% erhöht wird, bevor 80 mol-% der im Gesamtverlauf der thermischen Behandlung insgesamt freigesetzten Gesamtmenge M^{A} an Ammoniak freigesetzt worden sind.

9. Verfahren nach einem der Ansprüche 1 bis 8, **dadurch gekennzeichnet, dass** der Gehalt der Atmosphäre A an molekularem Sauerstoff, spätestens dann, wenn 98 mol% der im Gesamtverlauf der thermischen Behandlung insgesamt freigesetzten Gesamtmenge M^{A} an Ammoniak freigesetzt worden sind, auf einen Wert von 0,6 bis 4 Vol.% erhöht wird.

10. Verfahren nach einem der Ansprüche 1 bis 9, **dadurch gekennzeichnet, dass** der Gehalt der Atmopshäre A an molekularem Sauerstoff, spätestens dann, wenn 98 mol% der im Gesamtverlauf der thermischen Behandlung insgesamt freigesetzten Gesamtmenge M^{A} an Ammoniak freigesetzt worden sind, auf einen Wert von 1 bis 3 Vol.% erhöht wird.

11. Verfahren nach einem der Ansprüche 1 bis 10, **dadurch gekennzeichnet, dass** sich der Calcinationstemperaturbereich auf den Bereich von 380 bis 430°C erstreckt.

12. Verfahren nach einem der Ansprüche 1 bis 11, **dadurch gekennzeichnet, dass** nach beendeter Calcination das innige Trockengemisch innerhalb eines Zeitraums von ≤5 h auf eine Temperatur ≤100°C abgekühlt wird.

13. Verfahren nach Anspruch 12, **dadurch gekennzeichnet, dass** die Abkühlung bis wenigstens auf eine Temperatur von 350°C in einer Atmosphäre A erfolgt, deren Gehalt an molekularem Sauerstoff ≤5 Vol.% beträgt.

14. Verfahren nach einem der Ansprüche 1 bis 13, **dadurch gekennzeichnet, dass** der Ammoniakgehalt der Atmosphäre A im Verlauf der thermischen Behandlung ein Maximum durchläuft, das ≤10 Vol.-% beträgt.

15. Verfahren nach Anspruch 14, **dadurch gekennzeichnet, dass** die Atmosphäre A ihren maximalen Ammoniakgehalt erreicht, bevor das innige Trockengemisch den Calcinationstemperaturbereich erreicht hat.

16. Verfahren nach einem der Ansprüche 1 bis 15, **dadurch gekennzeichnet, dass** die katalytisch aktive Multimetalloxidmasse der allgemeinen Stöchiometrie I
Mo₁₂VₐX¹_{b}X²_{c}X³_{d}X⁴ₑX⁵_{f}X⁶_{g}Oₙ (I),
genügt, in der die Variablen folgende Bedeutung haben:
X¹ = W, Nb, Ta, Cr und/oder Ce,
X² = Cu, Ni, Co, Fe, Mn und/oder Zn,
X³ = Sb und/oder Bi,
X⁴ = einer oder mehrere Alkalimetalle,
X⁵ = eines oder mehrere Erdalkalimetalle,
X⁶ = Si, Al, Ti und/oder Zr,
a = 1 bis 6,
b = 0,2 bis 4,
c = 0,5 bis 18,
d = 0 bis 40,
e = 0 bis 2,
f = 0 bis 4,
g = 0 bis 40 und
n = eine Zahl, die durch die Wertigkeit und Häufigkeit der von Sauerstoff verschiedenen Elemente in I bestimmt wird.

17. Verfahren nach einem der Ansprüche 1 bis 16, **dadurch gekennzeichnet, dass** die katalytisch aktive Multimetalloxidmasse eine solche der allgemeinen Formel III,
[A]ₚ[B]_{q}[C]ᵣ (III)
ist, in der die Variablen folgende Bedeutung haben:
A = Mo₁₂VₐX¹_{b}X²_{c}X³_{d}X⁴ₑX⁵_{f}X⁶_{g}Oₓ,
B = X⁷₁CuₕHᵢO_{y},
C = X⁸₁SbⱼHₖO_{z},
X¹ = W, Nb, Ta, Cr und/oder Ce,
X² = Cu, Ni, Co, Fe, Mn und/oder Zn,
X³ = Sb und/oder Bi,
X⁴ = Li, Na, K, Rb, Cs und/oder H,
X⁵ = Mg, Ca, Sr und/oder Ba,
X⁶ = Si, Al, Ti und/oder Zr,
X⁷ = Mo, W, V, Nb und/oder Ta,
X⁸ = Cu, Ni, Zn, Co, Fe, Cd, Mn, Mg, Co, Sr und/oder Ba,
a = 1 bis 8,
b = 0,2 bis 5,
c = 0 bis 23,
d = 0 bis 50,
e = 0 bis 2,
f = 0 bis 5,
g = 0 bis 50,
h = 0,3 bis 2,5
i = 0 bis 2,
j = 0,1 bis 50,
k = 0 bis 50,
x, y, z = Zahlen, die durch die Wertigkeit und Häufigkeit der von Sauerstoff verschiedenen Elemente in A, B, C bestimmt werden,
p, q = positive Zahlen,
r = 0 oder eine positive Zahl, wobei das Verhältnis p/(q+r) = 20:1 bis 1:20, und für den Fall, dass r eine positive Zahl ist, das Verhältnis q/r = 20:1 bis 1:20 beträgt,
die den Anteil [A]ₚ, in Form dreidimensional ausgedehnter Bereiche A der chemischen Zusammensetzung
A: M0₁₂VₐX¹_{b}X²_{c}X³_{d}X⁴ₑX⁵_{f}X⁶_{g}Oₓ,
den Anteil [B]q in Form dreidimensional ausgedehnter Bereiche B der chemischen Zusammensetzung
B: X⁷₁CuₕHᵢOy und
den optionalen Anteil [C]ᵣ in Form dreidimensional ausgedehnter Bereiche C der chemischen Zusammensetzung
C: X⁸₁SbⱼHₖO_{z}
enthält, wobei die Bereiche A, B und gegebenenfalls C relativ zueinander wie in einem Gemisch aus feinteiligem A, feinteiligem B und gegebenenfalls feinteiligem C verteilt sind.

18. Verfahren nach einem der Ansprüche 1 bis 17, **dadurch gekennzeichnet, dass** die thermische Behandlung in einem von einem Gasstrom durchströmten Drehrohrofen durchgeführt wird.

19. Verfahren nach Anspruch 18, **dadurch gekennzeichnet, dass** der Drehrohrofen diskontinuierlich betrieben wird.

20. Verfahren nach einem der Ansprüche 18 oder 19, **dadurch gekennzeichnet, dass** wenigstens eine Teilmenge des das Drehrohr durchströmenden Gasstroms im Kreis geführt wird.

21. Verfahren nach einem der Ansprüche 18 bis 20, **dadurch gekennzeichnet, dass** der Druck des das Drehrohr durchströmenden Gasstroms beim Verlassen des Drehrohres unterhalb des Umgebungsdrucks des Drehrohrs liegt.

22. Verfahren der heterogen katalysierten Partialoxidation von Acrolein zu Acrylsäure, **dadurch gekennzeichnet, dass** der eingesetzte Katalysator als Aktivmasse ein unmittelbares Verfahrensprodukt gemäß eines der Ansprüche 1 bis 21 aufweist.

23. Verfahren der heterogen katalysierten Partialoxidation von Methacrolein zu Methacrylsäure, **dadurch gekennzeichnet, dass** der eingesetzte Katalysator als Aktivmasse ein unmittelbares Verfahrensprodukt gemäß eines der Ansprüche 1 bis 21 aufweist.

24. Verfahren der heterogen katalysierten Partialoxidation von Propan zu Acrylsäure, **dadurch gekennzeichnet, dass** der eingesetzte Katalysator als Aktivmasse ein unmittelbares Verfahrensprodukt gemäß eines der Ansprüche 1 bis 21 aufweist.

## Claims

1. A process for the preparation of catalytically active multielement oxide materials which contain at least one of the elements Nb and W and the elements Mo, V and Cu, the molar fraction of the element Mo, based on the total amount of all elements other than oxygen in the catalytically active multielement oxide material, being from 20 to 80 mol%, the molar ratio of Mo contained in the catalytically active multielement oxide material to V, Mo/V contained in the catalytically active multielement oxide material being from 15:1 to 1:1, the corresponding molar ratio Mo/Cu being from 30:1 to 1:3 and the corresponding molar ratio Mo/(total amount of W and Nb) being from 80:1 to 1:4, in which an intimate dry blend also containing ammonium ions is prepared from starting compounds which contain the elemental constituents of the multielement oxide material, other than oxygen, as components and said dry blend is thermally treated at elevated temperatures in an atmosphere having a low content of molecular oxygen, at least a portion of the ammonium ions contained in the intimate dry blend being decomposed at ≥ 160°C with liberation of ammonia, wherein the thermal treatment is carried out as follows:
- the intimate dry blend is heated at a heating rate of ≤ 10°C/min to a decomposition temperature in the decomposition temperature range from 240°C to 360°C and is kept in this temperature range until at least 90 mol% of the total amount M^{A} of ammonia liberated altogether in the entire course of the thermal treatment of the intimate dry blend from the intimate dry blend at above 160°C have been liberated;
- the content of molecular oxygen in the atmosphere A in which the thermal treatment of the intimate dry blend takes place is reduced to ≤ 0.5% by volume no later than when the intimate dry blend has reached 230°C, and this low oxygen content is maintained until at least 20 mol% of the total amount M^{A} of ammonia liberated altogether in the entire course of the thermal treatment have been liberated;
- the intimate blend is taken at a rate of ≤ 10°C/min out of the decomposition temperature range and into the calcination temperature range of from 380 to 450°C no earlier than when ≥ 70 mol% of the total amount of M^{A} of ammonia liberated altogether in the entire course of the thermal treatment have been liberated and
- the content of molecular oxygen in the atmosphere A is increased to > 0.5 to 4% by volume no later than when 98 mol% of the total amount M^{A} of ammonia liberated altogether in the entire course of the thermal treatment have been liberated
and the intimate dry blend is calcined at this increased oxygen content of the atmosphere A in the calcination temperature range.

2. The process according to claim 1, wherein the content of ammonium ions in the intimate dry blend to be thermally treated is ≥ 5 mol% , based on the total molar content of elemental constituents of the subsequent catalytically active multielement oxide material, other than oxygen, in the intimate dry blend.

3. The process according to claim 1 or 2, wherein the temperature rate at which the intimate dry blend is heated to the decomposition temperature is ≤ 5°C/min.

4. The process according to any of claims 1 to 3, wherein the temperature rate at which the intimate dry blend is heated to the decomposition temperature is ≤ 3°C/min.

5. The process according to any of claims 1 to 4, wherein the decomposition temperature range is from 300 to 350°C.

6. The process according to any of claims 1 to 5, wherein the intimate dry blend is kept in the decomposition temperature range until at least 95 mol% of the total amount M^{A} of ammonia liberated altogether in the entire course of the thermal treatment of the intimate dry blend from the intimate dry blend at above 160°C have been liberated.

7. The process according to any of claims 1 to 6, wherein the content of molecular oxygen in the atmosphere A is ≤ 0.3% by volume no later than when the intimate dry blend has reached 230°C.

8. The process according to any of claims 1 to 7, wherein the content of molecular oxygen in the atmosphere A is increased to > 0.5 to 4% by volume before 80 mol% of the total amount M^{A} of ammonia liberated altogether in the entire course of the thermal treatment have been liberated.

9. The process according to any of claims 1 to 8, wherein the content of molecular oxygen in the atmosphere A is increased to 0.6 to 4% by volume no later than when 98 mol% of the total amount M^{A} of ammonia liberated altogether in the entire course of the thermal treatment have been liberated.

10. The process according to any of claims 1 to 9, wherein the content of molecular oxygen in the atmosphere A is increased to 1 to 3% by volume no later than when 98 mol% of the total amount M^{A} of ammonia liberated altogether in the entire course of the thermal treatment have been liberated.

11. The process according to any of claims 1 to 10, wherein the calcination temperature range is from 380 to 430°C.

12. The process according to any of claims 1 to 11, wherein, after calcination is complete, the intimate dry blend is cooled to ≤ 100°C in the course of a period of ≤ 5 hours.

13. The process according to claim 12, wherein the cooling is effected to at least 350°C in an atmosphere A whose content of molecular oxygen is ≤ 5% by volume.

14. The process according to any of claims 1 to 13, wherein the ammonia content of the atmosphere A passes through a maximum which is ≤ 10% by volume in the course of the thermal treatment.

15. The process according to claim 14, wherein the atmosphere A reaches its maximum ammonia content before the intimate dry blend has reached the calcination temperature range.

16. The process according to any of claims 1 to 15, wherein the catalytically active multimetal oxide material satisfies the stoichiometry I
Mo₁₂VₐX¹_{b}X²_{c}X³_{d}X⁴ₑX⁵_{f}X⁶gOₙ (I),
where:
X¹ is W, Nb, Ta, Cr and/or Ce,
X² is Cu, Ni, Co, Fe, Mn and/or Zn,
X³ is Sb and/or Bi,
X⁴ is one or more alkali metals,
X⁵ is one or more alkaline earth metals,
X⁶ is Si, Al, Ti and/or Zr,
a is from 1 to 6,
b is from 0.2 to 4,
c is from 0.5 to 18,
d is from 0 to 40,
e is from 0 to 2,
f is from 0 to 4,
g is from 0 to 40 and
n is a number which is determined by the valency and frequency of the elements other than oxygen in I.

17. The process according to any of claims 1 to 16, wherein the catalytically active multimetal oxide material is one of the formula III
[A]ₚ[B]_{q}[C]ᵣ (III)
where:
A is M0₁₂VₐX¹_{b}X²_{c}X³_{d}X⁴ₑX⁵_{f}X⁶_{g}O_{X},
B is X⁷₁CuₕHᵢO_{y},
C is X⁸₁SbⱼHₖO_{z},
X¹ is W, Nb, Ta, Cr and/or Ce,
X² is Cu, Ni, Co, Fe, Mn and/or Zn,
X³ is Sb and/or Bi,
X⁴ is Li, Na, K, Rb, Cs and/or H,
X⁵ is Mg, Ca, Sr and/or Ba,
X⁶ is Si, Al, Ti and/or Zr,
X⁷ is Mo, W, V, Nb and/or Ta,
X⁸ is Cu, Ni, Zn, Co, Fe, Cd, Mn, Mg, Co, Sr and/or Ba,
a is from 1 to 8,
b is from 0.2 to 5,
c is from 0 to 23,
d is from 0 to 50,
e is from 0 to 2,
f is from 0 to 5,
g is from 0 to 50,
h is from 0.3 to 2.5
i is from 0 to 2,
j is from 0.1 to 50,
k is from 0 to 50,
x, y and z are numbers which are determined by the valency and frequency of the elements other than oxygen in A, B and C,
p and q are positive numbers and
r is 0 or a positive number, the ratio p/(q+r) being from 20:1 to 1:20 and, where r is a positive number, the ratio q/r being from 20:1 to 1:20,
which contains the moiety [A]p in the form of three-dimensional regions A having the chemical composition
A: Mo₁₂VₐX¹_{b} X²_{c}X³_{d}X⁴ₑX⁵_{f}X⁶_{g}Oₓ,
the moiety [B]q in the form of three-dimensional regions B having the chemical composition
B: X⁷₁CuₕHᵢO_{y} and
the optional moiety [C]ᵣ in the form of three-dimensional regions C having the chemical composition
C: X⁸₁SbⱼHₖO_{z},
the regions A, B and, if desired, C being distributed relative to one another as in a mixture of finely divided A, finely divided B and, if desired, finely divided C.

18. The process according to any of claims 1 to 17, wherein the thermal treatment is carried out in a rotary tube furnace through which a gas stream flows.

19. The process according to claim 18, wherein the rotary tube furnace is operated batchwise.

20. The process according to either of claims 18 and 19, wherein at least a portion of the gas stream running through the rotary tube is circulated.

21. The process according to any of claims 18 to 20, wherein the pressure of the gas stream flowing through the rotary tube on leaving the rotary tube is below the ambient pressure of the rotary tube.

22. A process for the heterogeneously catalyzed partial oxidation of acrolein to acrylic acid, wherein the catalyst used comprises, as active material, a direct product of a process according to any of claims 1 to 21.

23. A process for the heterogeneously catalyzed partial oxidation of methacrolein to methacrylic acid, wherein the catalyst used comprises, as active material, a direct product of a process according to any of claims 1 to 21.

24. A process for the heterogeneously catalyzed partial oxidation of propane to acrylic acid, wherein the catalyst used comprises, as active material, a direct product of a process according to any of claims 1 to 21.

## Revendications

1. Procédé de fabrication de masses d'oxyde de plusieurs éléments catalytiquement actives, qui contiennent au moins un des éléments Nb et W, ainsi que les éléments Mo, V et Cu, la proportion molaire de l'élément Mo par rapport à la quantité totale de tous les éléments différents de l'oxygène de la masse d'oxyde de plusieurs éléments catalytiquement active étant de 20 % en moles à 80 % en moles, le rapport molaire entre le Mo contenu dans la masse d'oxyde de plusieurs éléments catalytiquement active et le V contenu dans la masse d'oxyde de plusieurs éléments catalytiquement active, Mo/V, étant de 15:1 à 1:1, le rapport molaire correspondant Mo/Cu étant de 30:1 à 1:3 et le rapport molaire correspondant Mo/(quantité totale de W et Nb) étant de 80:1 à 1:4, selon lequel un mélange intime sec contenant également des ions ammonium est fabriqué à partir de composés de départ qui contiennent les constituants élémentaires différents de l'oxygène de la masse d'oxyde de plusieurs éléments en tant que constituants, et celui-ci est traité thermiquement dans une atmosphère pauvre en oxygène moléculaire à température élevée, au moins une partie des ions ammonium contenus dans le mélange intime sec étant décomposés en libérant de l'ammoniac à des températures ≥ 160 °C, **caractérisé en ce que** le traitement thermique a lieu de la manière suivante :
- le mélange intime sec est porté avec un taux de chauffe ≤ 10 °C/min à une température de décomposition dans la plage de températures de décomposition allant de 240 °C à 360 °C et maintenu dans cette plage de températures jusqu'à ce qu'au moins 90 % en moles de la quantité totale M^{A} d'ammoniac libérée au total à partir du mélange intime sec pendant la totalité du traitement thermique du mélange intime sec à des températures supérieures à 160 °C ait été libérée ;
- au plus tard lorsque le mélange intime sec a atteint la température de 230 °C, la teneur de l'atmosphère A, dans laquelle le traitement thermique du mélange intime sec a lieu, en oxygène moléculaire est réduite à une valeur ≤ 0,5 % en volume et cette teneur en oxygène inférieure est maintenue jusqu'à ce qu'au moins 20 % en moles de la quantité totale M^{A} d'ammoniac libérée au total pendant la totalité du traitement thermique ait été libérée ;
- au plus tôt lorsque ≥ 70 % en moles de la quantité totale M^{A} d'ammoniac libérée au total pendant la totalité du traitement thermique a été libérée, le mélange intime sec est porté avec un taux ≤ 10 °C/min hors de la plage de températures de décomposition et dans la plage de températures de calcination de 380 à 450 °C, et
- au plus tard lorsque 98 % en moles de la quantité totale M^{A} d'ammoniac libérée au total pendant la totalité du traitement thermique a été libérée, la teneur de l'atmosphère A en oxygène moléculaire est augmentée dans la plage allant de > 0,5 % en volume à 4 % en volume,
et le mélange intime sec est calciné à cette teneur en oxygène élevée de l'atmosphère A dans la plage de températures de calcination.

2. Procédé selon la revendication 1, **caractérisé en ce que** la teneur du mélange intime sec à traiter thermiquement en ions ammonium, par rapport à la teneur molaire totale du mélange intime sec en constituants élémentaires différents de l'oxygène de la masse d'oxyde de plusieurs éléments ultérieurement catalytiquement active, est ≥ 5 % en moles.

3. Procédé selon la revendication 1 ou 2, **caractérisé en ce que** le taux de chauffe avec lequel le mélange intime sec est porté à la température de décomposition est ≤ 5 °C/min.

4. Procédé selon l'une quelconque des revendications 1 à 3, **caractérisé en ce que** le taux de chauffe avec lequel le mélange intime sec est porté à la température de décomposition est ≤ 3 °C/min.

5. Procédé selon l'une quelconque des revendications 1 à 4, **caractérisé en ce que** la plage de températures de décomposition s'étend dans la plage allant de 300 à 350 °C.

6. Procédé selon l'une quelconque des revendications 1 à 5, **caractérisé en ce que** le mélange intime sec est maintenu dans la plage de températures de décomposition jusqu'à ce qu'au moins 95 % en moles de la quantité totale M^{A} d'ammoniac libérée au total à partir du mélange intime sec pendant la totalité du traitement thermique du mélange intime sec à des températures supérieures à 160 °C ait été libérée.

7. Procédé selon l'une quelconque des revendications 1 à 6, **caractérisé en ce qu'**au plus tard lorsque le mélange intime sec a atteint la température de 230 °C, la teneur de l'atmosphère A en oxygène moléculaire est ≤ 0,3 % en volume.

8. Procédé selon l'une quelconque des revendications 1 à 7, **caractérisé en ce que** la teneur de l'atmosphère A en oxygène moléculaire est augmentée à une valeur allant de > 0,5 % en volume à 4 % en volume avant que 80 % en moles de la quantité totale M^{A} d'ammoniac libérée au total pendant la totalité du traitement thermique ait été libérée.

9. Procédé selon l'une quelconque des revendications 1 à 8, **caractérisé en ce que** la teneur de l'atmosphère A en oxygène moléculaire est augmentée à une valeur allant de 0,6 à 4 % en volume au plus tard lorsque 98 % en moles de la quantité totale M^{A} d'ammoniac libérée au total pendant la totalité du traitement thermique a été libérée.

10. Procédé selon l'une quelconque des revendications 1 à 9, **caractérisé en ce que** la teneur de l'atmosphère A en oxygène moléculaire est augmentée à une valeur allant de 1 à 3 % en volume au plus tard lorsque 98 % en moles de la quantité totale M^{A} d'ammoniac libérée au total pendant la totalité du traitement thermique a été libérée.

11. Procédé selon l'une quelconque des revendications 1 à 10, **caractérisé en ce que** la plage de températures de calcination s'étend dans la plage allant de 380 à 430 °C.

12. Procédé selon l'une quelconque des revendications 1 à 11, **caractérisé en ce qu'**après la fin de la calcination, le mélange intime sec est refroidi à une température ≤ 100 °C en une durée ≤ 5 h.

13. Procédé selon la revendication 12, **caractérisé en ce que** le refroidissement a lieu au moins jusqu'à une température de 350 °C dans une atmosphère A dont la teneur en oxygène moléculaire est ≤ 5 % en volume.

14. Procédé selon l'une quelconque des revendications 1 à 13, **caractérisé en ce que** la teneur en ammoniac de l'atmosphère A au cours du traitement thermique passe par un maximum qui est ≤ 10 % en volume.

15. Procédé selon la revendication 14, **caractérisé en ce que** l'atmosphère A atteint sa teneur en ammoniac maximale avant que le mélange intime sec n'atteigne la plage de températures de calcination.

16. Procédé selon l'une quelconque des revendications 1 à 15, **caractérisé en ce que** la masse d'oxyde de plusieurs métaux catalytiquement active satisfait la stoechiométrie générale I
Mo₁₂VₐX¹_{b}X²_{c}X³_{d}X⁴ₑX⁵_{f}X⁶_{g}Oₙ (I),
dans laquelle les variables ont la signification suivante :
X¹ = W, Nb, Ta, Cr et/ou Ce,
X² = Cu, Ni, Co, Fe, Mn et/ou Zn,
X³ = Sb et/ou Bi,
X⁴ = un ou plusieurs métaux alcalins,
X⁵ = un ou plusieurs métaux alcalino-terreux,
X⁶ = Si, Al, Ti et/ou Zr,
a = 1 à 6,
b = 0,2 à 4,
c = 0,5 à 18,
d = 0 à 40,
e = 0 à 2,
f = 0 à 4,
g = 0 à 40 et
n = un nombre déterminé par la valence et la fréquence des éléments différents de l'oxygène dans I.

17. Procédé selon l'une quelconque des revendications 1 à 16, **caractérisé en ce que** la masse d'oxyde de plusieurs métaux catalytiquement active est de formule III
[A]ₚ[B]_{q}[C]ᵣ (III),
dans laquelle les variables ont la signification suivante :
A = Mo₁₂VₐX¹_{b}X²_{c}X³_{d}X⁴ₑX⁵_{f}X⁶_{g}Oₓ,
B = X⁷ ₁CuₕHᵢO_{y},
C = X⁸₁SbⱼHₖO_{z},
X¹ = W, Nb, Ta, Cr et/ou Ce,
X² = Cu, Ni, Co, Fe, Mn et/ou Zn,
X³ = Sb et/ou Bi,
X⁹ = Li, Na, K, Rb, Cs et/ou H,
X⁵ = Mg, Ca, Sr et/ou Ba,
X⁶ = Si, Al, Ti et/ou Zr,
X⁷ = Mo, W, V, Nb et/ou Ta,
X⁸ = Cu, Ni, Zn, Co, Fe, Cd, Mn, Mg, Co, Sr et/ou Ba,
a = 1 à 8,
b = 0,2 à 5,
c = 0 à 23,
d = 0 à 50,
e = 0 à 2,
f = 0 à 5,
g = 0 à 50,
h = 0,3 à 2,5,
i = 0 à 2,
j = 0,1 à 50,
k = 0 à 50,
x, y, z = nombres déterminés par la valence et la fréquence des éléments différents de l'oxygène dans A, B, C,
p, q = nombres positifs,
r = 0 ou un nombre positif, avec le rapport p/(q+r) = 20:1 à 1:20 et, si r est un nombre positif, le rapport q/r = 20:1 à 1:20,
qui contient la fraction [A]p sous la forme de domaines A étendus tridimensionnellement de composition chimique
A : Mo₁₂VₐX¹_{b}X²_{c}X³_{d}X⁴X⁵_{f}X⁶_{g}Oₓ,
la fraction [B]_{q} sous la forme de domaines B étendus tridimensionnellement de composition chimique
B = X⁷₁CuₕHᵢO_{y} et
la fraction optionnelle [C]ᵣ sous la forme de domaines C étendus tridimensionnellement de composition chimique
C : X⁸₁SbⱼHₖO_{z},
les domaines A, B et éventuellement C étant répartis les uns par rapport aux autres sous la forme d'un mélange de A finement divisé, B finement divisé et éventuellement C finement divisé.

18. Procédé selon l'une quelconque des revendications 1 à 17, **caractérisé en ce que** le traitement thermique est réalisé dans un four tubulaire rotatif traversé par un courant gazeux.

19. Procédé selon la revendication 18, **caractérisé en ce que** le four tubulaire rotatif est exploité de manière discontinue.

20. Procédé selon l'une quelconque des revendications 18 ou 19, **caractérisé en ce qu'**au moins une partie du courant gazeux traversant le tube rotatif est mise en circulation dans un circuit.

21. Procédé selon l'une quelconque des revendications 18 à 20, **caractérisé en ce que** la pression du courant gazeux traversant le tube rotatif à la sortie du tube rotatif se situe en dessous de la pression ambiante du tube rotatif.

22. Procédé d'oxydation partielle sous catalyse hétérogène d'acroléine en acide acrylique, **caractérisé en ce que** le catalyseur utilisé comprend en tant que masse active un produit direct du procédé selon l'une quelconque des revendications 1 à 21.

23. Procédé d'oxydation partielle sous catalyse hétérogène de méthacroléine en acide méthacrylique, **caractérisé en ce que** le catalyseur utilisé comprend en tant que masse active un produit direct du procédé selon l'une quelconque des revendications 1 à 21.

24. Procédé d'oxydation partielle sous catalyse hétérogène de propane en acide acrylique, **caractérisé en ce que** le catalyseur utilisé comprend en tant que masse active un produit direct du procédé selon l'une quelconque des revendications 1 à 21.
